# EUROPEAN PATENT APPLICATION

(11) **EP 3 824 894 A1**
(43) Date of publication of application: **26.05.2021**
(21) Application number: 19831381.9
(22) Date of filing: 02.07.2019
(51) Int. Cl.: A61K 35/545, A61K 35/28, A61K 48/00, A61P 25/28, A61P 21/00

(54) **GENE AND CELL THERAPY PRODUCT USING CELL FUSION TECHNOLOGY AND USE THEREOF**

(30) Priority: 02.07.2018 KR 20180076719; 31.07.2018 KR 20180089538
(71) Applicant: Curamys Co., Ltd., Seoul 03080 (KR)
(72) Inventor: SUNG, Jung-Joon, Seoul 03080 (KR); KIM, Ki Yoon, Seoul 03080 (KR); JEON, Gye Sun, Seoul 06299 (KR)
(74) Representative: Vossius & Partner Patentanwälte Rechtsanwälte mbB
(86) International application number: PCT/KR2019/008081
(87) International publication number: WO 2020/009444

(57) **Abstract**

The present invention relates to gene and cell therapy product using a cell fusion technology and use thereof, and more particularly, cells overexpressing hemagglutinin neuraminidase (HN) and fusion (F) proteins have effects of enhancing cell fusion with other cells by HN and F proteins, restoring damaged or dying cells or cells having gene abnormality to normal cells through the cell fusion. Therefore, a vector including genes encoding the HN and F proteins of the present invention or a cell transformed with the vector can be used as an active ingredient for prevention or treatment of neurodegenerative diseases or nerve diseases or degenerative muscular diseases or muscle diseases.

## Description

### [Technical Field]

The present invention relates to gene and cell therapy product using a cell fusion technology and use thereof, and more particularly, to gene and cell therapeutic agents using a cell fusion technology capable of enhancing cell fusion with other cells by transducing hemagglutinin neuraminidase (HN) and fusion (F) genes into cells and overexpressing the transduced cells, restoring cell damage through cell fusion with damaged or dying cells or cells having gene abnormality and delivering normal genes and use thereof in diseases related to cell damage.

### [Background Art]

Generally, diseases and aging are progressed by cell damage and apoptosis. The common diseases that cause the cell damage and the apoptosis include neurodegenerative diseases, muscular dystrophy, and the like.

With the rapid increase in the elderly population, neurodegenerative diseases including damages of the brain, the spine and the peripheral nerves have been continuously increased. The causes of the neurodegenerative diseases are not clear yet. In addition, a pathological mechanism of each neurodegenerative disease is known to act a little different mechanism.

For example, amyotrophic lateral sclerosis (ALS), spinal muscular atrophy, and Kennedy's disease (spinal bulbar muscular atrophy) and the like are diseases in which only motor nerve cells are selectively killed and only the motor functions degenerate. As a result, these diseases may show symptoms of quadriplegia and are also called motor neuron disease.

Meanwhile, unlike the motor neuron disease, Alzheimer's disease, Parkinson's disease and the like are known as representative neurodegenerative diseases in which brain nerve cells are lost. In the case of Alzheimer's disease, general brain atrophy is seen due to nerve cell loss. These brain pathology findings are mainly localized in the hippocampus and olfactory cortex which are the major brain zones responsible for memory at the early stage of the disease and gradually spread to the entire brain through the parietal lobe and frontal lobe. As the brain pathology invaded-zone expands, memory loss mainly appears at the early stage and the clinical symptoms diversify and become more and more severe with gradual progress of the disease. Parkinson's disease is caused by the loss of dopaminergic neuron in the substantia nigra (SN) located in the midbrain. The loss of dopaminergic neuron causes severe dopamine reduction in the striatum, resulting in impaired motor control function by the extrapyramidal system including basal ganglia, thalamus, and motor cortex.

Since various pathological mechanisms work in this way, it is difficult to treat the disease with a therapeutic agent which acts on any one mechanism. For this reason, almost all clinical trials so far have failed, and there is no fundamental therapeutic agent. Particularly, the ALS-related therapeutic agents which are approved by the FDA in US are almost not existent except for riruzole, which inhibits excess glutamic acid that destroys moter neurons, and edaravone, which inhibits oxidative stress damage caused by free radicals, and these have only an effect of the prolonged survival for about 3 months or slightly slowing deterioration of the physical function respectively. In the case of Alzheimer's disease, there are Aricept, Exelon, Namenda, Razadyne and the like that activate acetylcholine of a neurotransmitter which helps the action of brain nerve cells as US FDA-approved therapeutic agents. However, these also only minimize brain cell damage to alleviate the symptoms or slow the progress but are not a satisfactory therapeutic agent. In the case of Parkinson's disease as well, levodopa as a dopamine agonist is used as the basis of treatment, but its long-term effect is limited. In the case of other neurodegenerative diseases as well, there is no therapeutic agent for complete cure. Hence, there is an urgent need for a therapeutic agent which acts on a broader mechanism and has a remarkable effect, and neurodegenerative diseases can be called diseases which desperately require new treatment methods including stem cell therapy.

Currently, various therapeutic methods, such as cell transplantation and the administration of drugs to improve the symptoms, are proposed to treat the neurodegenerative diseases, and especially recently, there is attention on cell therapy. However, a conventional cell therapy technology aims to insert health cells (alternatively, stem cells) into a diseased region to replace dead cells or improve an ambient environment of the dying cells to regenerate the dying cells, but the attempt has no effect or a slight effect in many preclinical or clinical trials. Further, in the case of neuronal cells, it is very important to form a neural circuit in terms of a function unlike other organs, and thus, it is very difficult for the cell supplied from the outside to be differentiated into the neuronal cells to restore the existing neural circuit as it is. Accordingly, in addition to the conventional methods, it is urgent to develop a new therapeutic method for reducing or protecting neuronal cell damage.

Meanwhile, muscles are essential tissues which support the body and maintain life phenomena. In the muscle, myoblasts differentiate to form multinucleated myotubes and myocytes, and the death of myoblasts and myocytes is a cause of various diseases . Diseases in which myocytes are lost by gene mutations or various causes have been reported, but there is no fundamental therapeutic agent to date. These muscle diseases vary in symptoms and severity depending on the type and may have a complex form due to problems in the nervous system such as neurons or spinal cord.

As an example, muscular dystrophy is a disease in which muscle cells are destroyed and is caused by genetic abnormalities, and the symptoms thereof begin with weakening of strength and difficulty in getting up, gradually worsen, and may lead to paralysis of respiratory muscles and death. There are various types of muscular dystrophy such as Duchenne muscular dystrophy (DMD) , Becker muscular dystrophy (BMD) , and limb girdle muscular dystrophy, and the symptoms thereof also differ depending on the type. The Duchenn muscular dystrophy (DMD) and Backer muscular dystrophy (BMD) are caused by abnormality of a dystrophin gene existing in an X chromosome and about 1/3 thereof is caused by natural mutation and the rest is caused by sex-linkage. Both the DMD and the BMD are caused by the abnormality of the same gene, but the DMD is called a case in which a phenotype is severe due to frame-shift mutation and the like. In the case of the DMD, since the course of the disease is poor, in 9 to 13 years of age, almost all patients are unable to walk and may be accompanied by cognitive decline as well as weakness of muscles accompanied by cardiomyopathy and respiratory distress to lead to death. In the case of the DMD, recently, a method of attempting treatment by exon skipping has emerged. Since the exon skipping targets a splicing enhancer sequence of exon 51 of a dystrophin gene and has a principle that restores only a reading frame converting the severe mutation to a less severe gene mutant, the exon skipping may not be a complete treatment alternative and is not a treatment method for targeting all DMDs.

As described above, as the disease progresses, patients with muscle diseases cannot live their daily lives alone, and there is no fundamental therapeutic agent. Therefore, there is an urgent need for a new treatment method which can effectively treat muscle diseases caused by muscle cell damage or death.

Therefore, the present inventors made efforts to develop a therapeutic agent for cell damage-related diseases including neurodegenerative diseases, degenerative muscular diseases, and the like, and as a result, found that cells overexpressing hemagglutinin neuraminidase (HN) and fusion (F) proteins have enhanced cell fusion with other cells by the HN and F proteins and high ability of restoring cell damage in the damaged or dying cells or cells having gene abnormality and delivering normal genes. In addition, the present inventors found that cell fusion technology using HN and F proteins can be usefully used to treat neurodegenerative diseases or neurological diseases or degenerative muscular disease or muscular diseases, and then completed the present invention.

### [Citation List]

### [Patent Literature]

Korean Publication Patent No. 10-2008-0026786
Korean Publication Patent No. 10-2006-0119064

### [Non Patent Literature]

Annemieke Aartsma-Rus et al., Development of Exon Skipping Therapies for Duchenne Muscular Dystrophy: A Critical Review and a Perspective on the Outstanding Issues, Nucleic Acid Ther. 2017 Oct 1; 27(5): 251-259

### [Summary of Invention]

### [Technical Problem]

An object of the present invention is to provide gene and cell therapeutic agents and their uses in diseases related to cell damage, more particularly, neurodegenerative diseases or neurological diseases or degenerative muscular diseases or muscular diseases.

### [Solution to Problem]

In order to achieve the above object, the present invention provides a pharmaceutical composition for prevention or treatment of neurodegenerative diseases or neurological diseases or degenerative muscular diseases or muscular diseases comprising a vector including genes encoding hemagglutinin neuraminidase (HN) and fusion (F) proteins as an active ingredient.

In addition, the present invention provides a pharmaceutical composition for prevention or treatment of neurodegenerative diseases or neurological diseases or degenerative muscular diseases or muscular diseases comprising a cell transformed with a vector including genes encoding hemagglutinin neuraminidase (HN) and fusion (F) proteins as an active ingredient.

In addition, the present invention provides a method for preventing or treating neurodegenerative diseases or neurological diseases or degenerative muscular diseases or muscular diseases comprising: administering a therapeutically effective dose of a vector including genes encoding hemagglutinin neuraminidase (HN) and fusion (F) proteins to a subject.

In addition, the present invention provides a method for preventing or treating neurodegenerative diseases or neurological diseases or degenerative muscular diseases or muscular diseases comprising: administering a therapeutically effective dose of a cell transformed with a vector including genes encoding hemagglutinin neuraminidase (HN) and fusion (F) proteins to a subject.

In addition, the present invention provides use of a vector including genes encoding hemagglutinin neuraminidase (HN) and fusion (F) proteins in a pharmaceutical composition for prevention or treatment of neurodegenerative diseases or neurological diseases or degenerative muscular diseases or muscular diseases.

In addition, the present invention provides use of a cell transformed with a vector including genes encoding hemagglutinin neuraminidase (HN) and fusion (F) proteins in a pharmaceutical composition for prevention or treatment of neurodegenerative diseases or neurological diseases or degenerative muscular diseases or muscular diseases.

### [Advantageous Effects of Invention]

The present invention confirmed that the cells overexpressing the HN/F proteins promote cell fusion with other cells by the HN/F proteins, and thus, normal genes and transcriptional regulators in the HN/F proteins overexpressed cells are transferred into the nucleus of damaged cells through the fusion with the damaged cells, thereby regulating the expression of the gene involved in cell repair in damaged cells or restoring normal proteins through the normal gene transfer, so restoring cell damage. In addition, the present invention confirmed that in various disease models related to cell damage, the cells overexpressing the HN/F proteins are fused with damaged cells or cells induced gene abnormality, thereby recovering to normal cells.

Therefore, cell fusion technology using hemagglutinin neuraminidase (HN) and fusion (F) proteins can be used to restore cell damage and introduce normal genes. Accordingly, a vector including genes encoding hemagglutinin neuraminidase (HN) and fusion (F) proteins or a cell transformed with the vector can be used to treat diseases related to cell damage, and more particularly, neurodegenerative diseases or neurological diseases or degenerative muscular diseases or muscular diseases.

### [Brief Description of Drawings]

FIG. 1 illustrates an effect of inhibiting cell death of a dying NSC34 motorneuron cell line by cell fusion with hATMSCs according to an exemplary embodiment of the present invention;
FIG. 2 illustrates an expression analysis of Bax and Bcl-xL in a NSC34 cell group which fused with hATMSCs according to the exemplary embodiment of the present invention;
FIG. 3 is a schematic diagram for preparing pcDNA3.1 expression vectors inserted with HN and F according to the exemplary embodiment of the present invention;
FIG. 4 illustrates an analysis of expression of HN and F mRNAs in HN/F-hATMSCs according to the exemplary embodiment of the present invention;
FIG. 5 illustrates an imaging analysis of expression of HN and F proteins in HN/F-hATMSCs according to the exemplary embodiment of the present invention;
FIG. 6 illustrates an immunophenotyping analysis of HN/F-hATMSCs according to the exemplary embodiment of the present invention;
FIG. 7 illustrates an analysis of cell fusion ability with a NSC34 motorneuron cell line in HN/F-hATMSCs according to the exemplary embodiment of the present invention;
FIG. 8 illustrates an analysis of expression of ChAT and CD105 in fused cells of the NSC34 motorneuron cell line and the HN/F-hATMSCs according to the exemplary embodiment of the present invention;
FIG. 9 illustrates a proteomic analysis result among the NSC34 motorneuron cell line, the dying NSC34 motorneuron cell line, and the HN/F-hATMSCs according to the exemplary embodiment of the present invention;
FIG. 10 illustrates a heatmap analysis result among the NSC34 motorneuron cell line, the dying NSC34 motorneuron cell line, and the HN/F-hATMSCs according to the exemplary embodiment of the present invention;
FIG. 11 illustrates an analysis of expression of genes DDB1, HMGB1, and MSH2 mRNAs related with cell repair in the NSC34 motorneuron cell line, the dying NSC34 motorneuron cell line, and the HN/F-hATMSCs according to the exemplary embodiment of the present invention;
FIG. 12 illustrates an apoptosis inhibitory effect in a dying C2C12 muscle myoblast cell line by cell fusion with HN/F-NSCs according to the exemplary embodiment of the present invention;
FIG. 13 illustrates an analysis of expression of HN and F mRNAs in HN/F-heLa according to an exemplary embodiment of the present invention;
FIG. 14 illustrates an analysis of cell fusion ability with a N2A neuroblastoma cell line in HN/F-HeLa according to the exemplary embodiment of the present invention;
FIG. 15 is a schematic diagram for preparing pcDNA3. 1-P2A expression vectors inserted with HN and F according to the exemplary embodiment of the present invention;
FIG. 16 illustrates an analysis of cell fusion ability with a C2C12 muscle myoblast cell line in HN/F-HeLa or F-P2A-HN-HeLa according to the exemplary embodiment of the present invention;
FIG. 17 illustrates confirming a TDP-43 binding motif which is a transcriptional factor (TF) in a promoter region of a gene DDB1 related with cell repair selected according to the exemplary embodiment of the present invention;
FIG. 18 is a schematic diagram for preparing a mouse DDB1 (mDDB1) specific binding promoter according to the exemplary embodiment of the present invention;
FIG. 19 illustrates an analysis of mDDB1 expression by TDP-43 in fused cells of a HeLa cell line transduced with GFP-TDP-43 and the N2A neuroblastoma cell line according to the exemplary embodiment of the present invention;
FIG. 20 illustrates an imaging analysis of translocation of TDP-43 from a nucleus of HeLa into a nucleus of the N2A neuroblastoma cell line in the fused cells of the HeLa cell line transduced with GFP-TDP-43 and the N2A neuroblastoma cell line according to the exemplary embodiment of the present invention;
FIG. 21 is a schematic diagram illustrating a cell damage restoration mechanism by HN/F protein overexpressed cells in a damaged cell;
FIG. 22 illustrates an apoptosis inhibitory effect of a dying Alzheimer's disease (AD) cell model by cell fusion with HN/F-hATMSCs according to the exemplary embodiment of the present invention;
FIG. 23 illustrates an apoptosis inhibitory effect of a dying Huntington's disease (HD) cell model by cell fusion with HN/F-hATMSCs according to the exemplary embodiment of the present invention;
FIG. 24 illustrates an apoptosis inhibitory effect of a dying heart failure disease cell model by cell fusion with HN/F-hATMSCs according to the exemplary embodiment of the present invention;
FIG. 25 illustrates an intra-spinal cord injection method for injecting HN/F-hATMSCs in a myotrophic lateral sclerosis (ALS) or Duchenne muscular dystrophy (DMD) disease mouse modelaccording to the exemplary embodiment of the present invention;
FIG. 26 illustrates an analysis of motion performance in a G93A SOD1 Tg mouse injected with HN/F-hATMSCs according to the exemplary embodiment of the present invention;
FIG. 27 illustrates an analysis results on the expression of dystrophin and CTGF in a DMD disease cell model prepared according to the exemplary embodiment of the present invention;
FIG. 28 illustrates an analysis results on the expression of dystrophin and CTGF in a DMD disease cell model treated with HN/F-hATMSCs according to the exemplary embodiment of the present invention;
FIG. 29 illustrates an analysis results on the dystrophin expression in skeletal muscle of mdx mouse that is a DMD disease animal model;
FIG. 30 illustrates an analysis results on the engraftment ability of HN/F-hATMSCs in skeletal muscle tissue of mdx mouse injected with HN/F-hATMSCs according to the exemplary embodiment of the present invention;
FIG. 31 illustrates an analysis results on the dystrophin expression in skeletal muscle tissue of mdx mouse injected with HN/F-hATMSCs in 3 weeks and 15 weeks after the injection according to the exemplary embodiment of the present invention; and
FIG. 32 illustrates an analysis results on the CTGF expression in skeletal muscle tissue of mdx mouse injected with HN/F-hATMSCs according to the exemplary embodiment of the present invention.

### [Description of Embodiments]

Hereinafter, the present invention will be described in detail by the following Examples.

The present invention provides a vector including genes encoding hemagglutinin neuraminidase (HN) and fusion (F) proteins.

In the present invention, the HN and F proteins may be derived from sendai virus, human immunodeficiency virus 1, Human parainfluenza virus, Human respiratory syncytial virus (HRSV), influenza virus or vesicular stomatitis virus, but is not limited thereto. Various types of HN and F proteins having various origins and/or full lengths and/or fragments may be used as long as these proteins may promote the fusion of cells, inhibit the death, and restore the damage to the damaged cells through the same or similar mechanism as or to these proteins (Eckert DM, Kim PS. Annu Rev Biochem. 2001;70:777-810.; Sapir A, Avinoam O, Podbilewicz B, Chernomordik LV. Dev Cell. 2008 Jan; 14 (1) :11-2 1.; Kouris NA, Schaefer JA, Hatta M, Freeman BT, Kamp TJ, Kawaoka Y, Ogle BM. Stem Cells Int. 2012;2012:414038) .

The hemagglutinin neuraminidase (HN) according to the present invention is a glucoprotein expressed in viruses and may exist as a single virus protein having activities of both hemagglutinin and neuraminidase or separate proteins, but is not limited thereto and all of the proteins may be included in the present invention as long as the proteins achieve an effect according to the present invention. Examples of the former may include those derived from paramyxoviruses such as a mumps virus, a sendai virus, a human parainfluenza virus, for example, a human parainfluenza virus type 1 (HPIV-1), a human parainfluenza virus type 2 (HPIV-2) or a human parainfluenza virus type 3 (HPIV-3), or an avian Newcastle disease virus and examples of the latter may include those derived from an influenza virus. These proteins and nucleic acid sequences are known, and for example, the hemagglutinin neuraminidase may refer to information in Enzyme entry (http: //enzyme.expasy. org) EC:3.2.1.18 and the protein sequences are derived from, for example, sendai viruses and published in GenBank accession no. AAB06288.1. In one embodiment according to the present invention, a sendai virus-derived HN single protein which may be prepared according to a method in Example 2 of the present invention is used.

Further, the fusion protein (F protein) is called a glucoprotein which is used in intercellular fusion or fusion/entry of virus into cells, endocytosis, and membrane trafficking. In one embodiment according to the present invention, virus-derived F proteins are used and divided into classes I, II, and III according to structural features. Examples of the class I include GP2 of ebola virus, Mo-55 of moloney murine leukemia virus (MoMuLv), gp41 of immunodeficiency virus HIV, simian virus (SIV) gp41, and the like. Examples of the class II include SFV E1, TBEV E, and the like. Examples of the class III include glycoprotein B (gB) of herpes simplex virus (HSV), gB of epstein-barr virus (EBV) , protein G of vesicular stomatitis virus (VSV), glycoprotein gp64 of beculovirus, and the like. Such fusion glycoproteins and nucleic acid sequences are published and for example, published in GenBank Accession no. AAC82300.1.

In the present invention, the genes encoding the HN and F proteins may be used as full-length and/or fragmental types. Particularly, wide-type gene sequences encoding the proteins disclosed in the present invention and fragments thereof include genes in which some of base sequences are artificially modified so that features such as expression in cells or stability of the proteins are advantageous, and naturally found genes in which some of base sequences are modified, or all fragments thereof.

In the present invention, the vector means a means for expressing a target gene in host cells. The vector includes elements for expressing the target gene and may include a replication origin, a promoter, an operator gene, a transcription terminator, and the like and may further include a suitable enzyme site (for example, a restrictive enzyme site) for introduction into a genome in the host cells and/or a selection marker for confirming successful introduction into the host cells, and/or a ribosome binding site (RBS) for translation to the protein, an internal ribosome entry site (IRES), and the like. The vector may further include a transcription regulator (for example, an enhancer and the like) in addition to the promoter.

Further, the vector may be a plasmid DNA, a recombinant vector, or other vectors, which are known in the art, and particularly, may be a linear DNA, a plasmid DNA, a non-viral recombinant vector, a viral recombinant vector, or an inducible gene expression vector system, and the viral recombinant vector may be retrovirus, adenovirus, adeno-associated virus, helper-dependent adenovirus, herpes simplex virus, lentivirus or vaccinia virus vectors, but is not limited thereto.

Further, the vector means an expression vector for gene therapy. The term "gene therapy" refers to a method of inserting a normal gene into cells having gene abnormality or proving a new function to normalize its function in order to treat various genetic diseases caused by the gene abnormality. Accordingly, in the present invention, the expression vector for gene therapy means an expression vector which may provide a new function through cell fusion between cells having gene abnormality and normal cells by transferring hemagglutinin neuraminidase (HN)/fusion (F) protein genes into normal cells in the body and therefore normalize its function.

In one embodiment of the present invention, the HN/F protein genes may be introduced into the cells according to the present invention by preparing primers which can specifically recognize the genes from known sequences, amplifying the genes through polymerase chain reaction using the primers, and introducing the amplified genes into the expression vector, as described below. The introduction method is known and for example, may include liposome-mediated transduction, calcium phosphate transduction, DEAE-dextran mediated transduction, positively charged lipid mediated transduction, electroporation, transduction using a phage system, infection using a virus, or the like, but the present invention is not limited thereto.

Further, the present invention provides a cell transformed with a vector including genes encoding hemagglutinin neuraminidase (HN) and fusion (F) proteins.

In the present invention, the term "transformation" means a phenomenon in which a genetic property of a living organism is changed by DNA given from the outside, that is, a genetic trait is changed while DNA is introduced to the cells thereof when DNA, a kind of nucleic acid extracted from cells in any system of the living organism is introduced into living cells in another system.

In the present invention, the transformed cells may be stem cells, progenitor cells, or animal cells, but the present invention is not limited thereto. Further, the stem cells may specifically be embryonic stem cells, adult stem cells, or induced pluripotent stem cells (iPS) but are not limited thereto. The stem cells include stem cells differentiated from the stem cells, for example, embryonic stem cells-derived mesenchymal stem cells, induced pluripotent stem cells-derived mesenchymal stem cells, and induced pluripotent stem cells-derived neural stem cells. Further, the cells may be autologous or allogeneic, or allogenic or xenogenic cells. Most preferably, since the cells are autologous-derived and derived from a recipient, there is no problem in immune response when a pharmaceutical composition is administered and there is an advantage in safety.

The embryonic stem cells are formed from the inner cell mass of the blastocyte at the initial stage of embryonic development, have the potential to differentiate into all cells (totipotent), have the advantage of being able to differentiate into any tissue cell, and are thus used in cell therapy studies.

The adult stem cells refer to undifferentiated stem cells found in the whole adult even after an embryonic development stage. The adult stem cells have a site-specific differentiation in which the cells themselves are differentiated according to characteristics of surrounding tissues. The adult stem cells may be derived from various adult cells, such as bone marrow, blood, brain, skin, fat, skeletal muscle, umbilical cord, cord blood, and the like. Specific examples thereof may include mesenchymal stem cells (MSC), skeletal muscle stem cells, hematopoietic stem cells, neuronal stem cells, adipose-derived stem cells, adipose-derived progenitor cells, vascular endothelial progenitor cells, and the like, but the present invention is not limited thereto.

In addition, the mesenchymal stem cells are adult stem cells obtained from the respective parts of the body that have already become adult, and refer to pluripotent or multipotent cells capable of differentiating into various cells, for example, adipocytes, motorneuron cells, and the like, as stem cells isolated from umbilical cord, cord blood, bone marrow, blood, brain, skin, fat, skeletal, muscle, nerve, periosteum, amniotic membrane or placenta. Further, the mesenchymal stem cells may be effectively engrafted from allogenic or xenogenic recipients without using immune inhibitors. The mesenchymal stem cells may be animal, particularly mammal, and more particularly human mesenchymal stem cells.

In one embodiment of the present invention, the mesenchymal stem cells are stem cells derived from adipose tissue. The mesenchymal stem cells derived from the adipose tissue have a practical advantage of being able to receive a large amount unlike bone marrow, amniotic fluid, and cord blood stem cells, about 1% of the adipocytes has been estimated as the stem cells, and recently, the adipose-derived stem cells are highly useful due to infinite supply ability when considering that a cosmetic surgery widely performed in the advanced countries is liposuction.

A process of obtaining the mesenchymal stem cells will be described as follows. The mesenchymal stem cells are isolated from mammals including humans or mice, preferably human mesenchymal stem cell sources, for example, adipose tissue, blood or bone marrow. Next, the cells are cultured in a suitable medium. In the culture process, the floating cells are removed and the cells attached to a culture plate are subcultured to finally obtain established mesenchymal stem cells.

In addition, a process of isolating and culturing a very small amount of mesenchymal stem cells in bone marrow and the like, is well known in the art, and for example, there is disclosed in US Patent Registration No. 5,486,359.

As the medium to be used in the above process, any medium generally used for culturing the stem cells may be used. Preferably, the medium is a medium containing serum (for example, fetal bovine serum, horse serum and human serum). The medium to be used in the present invention includes, for example, RPMI series, Eagles's MEM (Eagle's minimum essential medium, Eagle, H. Science 130:432(1959)), α-MEM (Stanner, C.P. et al., Nat. New Biol. 230:52(1971)), Iscove's MEM (Iscove, N. et al., J. Exp. Med. 147:923(1978)), 199 media (Morgan et al., Proc. Soc. Exp. Bio. Med., 73:1(1950)), CMRL 1066, RPMI 1640 (Moore et al., J. Amer. Med. Assoc. 199:519(1967)), F12 (Ham, Proc. Natl. Acad. Sci. USA 53:288(1965)), F10 (Ham, R.G. Exp. Cell Res. 29:515(1963)), DMEM (Dulbecco's Modified Eagle's medium, Dulbecco, R. et al., VirProcy 8:396(1959)), a mixture of DMEM and F12 (Barnes, D. et al., Anal. Biochem. 102:255(1980)), Way-mo, h's MB752/1 (Waymo, h, C. J. Natl. Cancer Inst. 22:1003(1959)), McCoy's5A (McCoy, T.A., etal., Proc. Soc. Exp. Biol. Med. 100:115(1959)) and MCDB series (Ham, R.G. et al., In Vitro 14:11(1978)), but the present invention is not limited thereto. In the media, other ingredients, for example, antibiotics, or antifungal agents (e.g., penicillin, streptomycin), glutamine, and the like may be included. A general description of the media and culture is disclosed in R. Ian Freshney, Culture of Animal Cells, Alan R. Liss, Inc., New York (1984), which is incorporated in this specification by reference.

The mesenchymal stem cells may be confirmed by, for example, a flow cytometry. The flow cytometry is performed by using a specific surface marker of the mesenchymal stem cells. In one embodiment of the present invention, the mesenchymal stem cells according to the present invention have CD29, CD44 and CD90 as markers and phenotypes of CD34, CD45 and HLA-DR as negative markers.

In addition, the inducible pluripotent stem cells can be prepared to multipotent stem cells such as embryonic stem cells by introducing four specific genes that induce dedifferentiation into somatic cells such as skin cells of a non-pluripotent adult, and then expressing the cells or extracting a dedifferentiation inducible protein prepared from the cells introduced with the four genes to inject the extracted protein into somatic cells again, which are called inducible multipotent stem cells or dedifferentiation stem cells. The inducible pluripotent stem cells are used for stem cell therapy using the inducible pluripotent stem cells, cell-based studies in disease model and drug development by producing inducible pluripotent stem cells and differentiating the stem cells in vitro by obtaining somatic cells from patients to study progress of various diseases, and the like. The induced pluripotent stem cells may be obtained according to known methods, for example, the method disclosed in Yu J et al. , (2007) Induced pluripotent stem cell lines derived from human somatic cells, Science, 318, 1917-1920 or Takahashi K et al., (2007) Induction of pluripotent stem cells from adult human fibroblasts by defined factors, Cell 131, 861-872. Further, the induced pluripotent stem cells include induced pluripotent stem cells-derived stem cells, for example, induced pluripotent stem cells-derived mesenchymal stem cells (iPSCs-MSCs) or induced pluripotent stem cells-derived neural stem cells (iPSCs-NSCs). In an embodiment of the present invention, the induced pluripotent stem cells-derived stem cells are induced pluripotent stem cells-derived neural stem cells.

The induced pluripotent stem cells-derived mesenchymal stem cells or induced pluripotent stem cells-derived neural stem cells may be obtained according to known methods, for example, the method disclosed in L.G. VILLA-DIAZ et al., (2012) Derivation of Mesenchymal Stem Cells from Human Induced Pluripotent Stem Cells Cultured on Synthetic Substrates, STEM CELLS 30, 1174-1181 or Hyun Soo Choi et al., (2014) Neural Stem Cells Differentiated From iPS Cells Spontaneously Regain Pluripotency, STEM CELLS 32, 2596-2604.

Further, the progenitor cell is a cell at a stage before the shape and function of a specific cell are established, and is a cell that can be differentiated into cells of a specific cell line or can be formed into a specific type of tissue, and means a cell having self-renewal, but an extremely limited differentiation. Endoderm progenitor cells, mesodermal progenitor cells, and ectoderm precursor cells are all included therein.

Further, the animal cell is a functional and structural basic unit originating from an animal including a human and may be included in the scope of the present invention if it is a cell originating from an animal including a human being (for example, a mammal such as a monkey, a dog, a goat, a pig, a mouse, and the like). Accordingly, the animal cells of the present invention are not limited thereto, but include epithelial cells, endothelial cells, muscular cells, germ cells, skin cells (e.g., fibroblasts, keratinocytes) , immune cells, cancer cells and the like. As specific examples, chinese hamster ovary (CHO) cells, mouse myeloma (NS0) cells, baby hamster kidney (BHK) cells, Sp2/0 (mouse myeloma) cells, human retinal cells, HUVEC cells, HMVEC cells, COS-1 cells, COS-7 cells, HeLa cells, HEK-293 cells, HepG-2 cells, HL-60 cells, IM-9 cells, Jurkat cells, MCF-7 cells or T98G cells may be exemplified, but the present invention is not limited thereto.

Further, the present invention provides a pharmaceutical composition for prevention or treatment of neurodegenerative diseases or neurological diseases or degenerative muscular diseases or muscular diseases comprising a vector including genes encoding hemagglutinin neuraminidase (HN) and fusion (F) proteins as an active ingredient.

In the present invention, the vector are the same as the description of the vector including genes encoding hemagglutinin neuraminidase (HN) and fusion (F) proteins, and the detailed description thereof refers to the above contents. Hereinafter, only a specific configuration of the pharmaceutical composition for prevention or treatment of neurodegenerative diseases or neurological diseases or degenerative muscular diseases or muscular diseases will be described.

Meanwhile, the present invention confirmed that the cells overexpressing the HN/F proteins promote cell fusion with other cells by the HN/F proteins, a normal gene is transferred and expressed through fusion with the damaged cells, and the expression of the gene for restoring the cell damage is regulated. In addition, the present invention confirmed that in various disease models related to cell damaged, the cells overexpressing the HN/F proteins are fused with damaged cells or cells induced gene abnormality, thereby recovering to normal cells. Therefore, the vector including the genes encoding the HN/F proteins may be usefully used as an active ingredient of composition for prevention or treatment of neurodegenerative diseases or neurological diseases or degenerative muscular diseases or muscular diseases.

In the present invention, the neurodegenerative diseases or neurological diseases or degenerative muscular diseases or muscular diseases are diseases related to cell damage.

The neurodegenerative diseases or neurological diseases may cause nerve cell damage, spinal nerve damage, peripheral nerve damage, or nerve cell death.

In addition, the neurodegenerative diseases or neurological diseases may divided into neurodegenerative diseases or neurological diseases that cause motor nerve cell damage or death, and neurodegenerative diseases or neurological diseases that cause brain nerve cell damage or death, spinal nerve damage, or peripheral nerve damage.

In order to improve or treat the neurodegenerative diseases or neurological diseases that cause motor nerve cell damage or death, preservation and recovery of motor nerve cells must be performed. As examples of such neurodegenerative diseases or neurological diseases, spinal muscular atrophy, Kennedy's disease (spinal bulbar muscular atrophy), amyotrophic lateral sclerosis (ALS), multiple sclerosis, primary lateral sclerosis or progressive bulbar palsy may be exemplified, but the present invention is not limited thereto.

The neurodegenerative diseases or neurological diseases that cause brain nerve cell damage or death, spinal nerve damage, or peripheral nerve damage can be prevented or treated through the recovery of peripheral nerves, the nerve cells of the damaged brain or spinal cord. As examples of such neurodegenerative diseases or neurological diseases, Alzheimer's disease (AD), dementia, multi-infarct dementia (mid), frontotemporal dementia, dementia with Lewy bodies, mild cognitive impairment, corticobasal degeneration, Parkinson's disease (PD), depression, metabolic brain disease, multiple system atrophy (MSA), Huntington's disease, progressive supranuclear palsy (PSP), epilepsy, dentatorubropallidoluysian atrophy (DRPLA), spinocerebellar ataxia, glaucoma, Stroke, brain ischemia, post-encephalitic parkinsonism, Tourette's syndrome, restless legs syndrome or attention deficit disorders with hyperactivity may be exemplified, but the present invention is not limited thereto.

The degenerative muscular diseases or muscular diseases may cause muscle cell damage or muscle cell death. As examples of such degenerative muscular diseases or muscular diseases, myopathy, congenital myopathy, congenital muscular dystrophy, Duchenne muscular dystrophy, Becker muscular dystrophy, Limb Girdle muscular dystrophy, Facioscapulohumeral muscular dystrophy, oculopharyngeal muscular atrophy, distal muscular dystrophy, Emery-Dreifuss muscular dystrophy, Myotonic dystrophy, Barth syndrome, heart failure, sarcopenia or X-linked dilated cardiomyopathy may be exemplified, but the present invention is not limited thereto.

The pharmaceutical composition according to the present invention may be formulated in a suitable form together with a pharmaceutically acceptable carrier which is generally used. The pharmaceutically acceptable carrier may include, for example, water, suitable oils, saline, parenteral administration carries such as aqueous glucose and glycol, and the like, and further include stabilizers and preservatives. The suitable stabilizer includes an antioxidant such as sodium hydrogen sulfite, sodium sulfite, or ascorbic acid. The suitable preservative includes benzalcohol chloride, methylor propyl-paraben, and chlorobutanol. In addition, the composition for cell therapy according to the present invention may suitably include suspending agents, solubilizers, stabilizers, isotonizing agents, preservatives, adsorption inhibitors, surfactants, diluents, excipients, pH adjusters, analgesic agents, buffers, antioxidants, and the like according to the administration method or the formulation, if necessary. The pharmaceutically acceptable carriers and formulations suitable for the present invention, including those exemplified above, are described in detail in the document [Remington's Pharmaceutical Sciences, latest edition].

The pharmaceutical composition of the present invention is formulated by using a pharmacologically acceptable carrier and/or excipient according to a method that may be easily performed by those skilled in the art to be prepared in a unit dosage form or prepared by intrusion into a multi-dose container.

Further, the pharmaceutical composition may also be administered by any device in which active ingredients may move to the target cell. The pharmaceutical composition of the present invention may be included with a therapeutically effective dose for treatment of diseases. In the present invention, the term "treatment' means reversing or mitigating one or more symptoms of a disease or a disorder to which the term is applied, or inhibiting or preventing the progress thereof, unless otherwise stated. The term "therapeutically effective dose" means an amount of an active ingredient or a pharmaceutical composition which induces a biological or medical response in tissue systems, animals, or humans which is considered by researchers, veterinarian, physician, or other clinicians, and includes an amount of inducing mitigation of symptoms of diseases or disorders to be treated. It is apparent to those skilled in the art that the active ingredients included in the pharmaceutical composition of the present invention are changed according to an effect.

Therefore, the optimal content of the pharmaceutical composition may be easily determined by those skilled in the art, and may be adjusted according to various factors including a type of disease, severity of the disease, contents of other ingredients contained in the composition, a type of formulation, and an age, a weight, a general health condition, a gender, and a diet of a patient, an administration time, a administration route, a secretion ratio of the composition, a treatment period, and simultaneously used drugs.

In one embodiment of the present invention, the pharmaceutical composition is administered intravenously or intrathecally.

It is important to include an amount capable of obtaining a maximum effect by a minimum amount without side effects by considering all of the factors. For example, the dose of the composition of the present invention may be 0.1 × 10⁵ to 1.0× 10⁸ cells/kg (body weight) and more preferably 0.5 × 10⁶ to 1.0× 10⁷ cells//kg (body weight) of a cell transformed with the vector including the genes encoding the HN and F proteins as active ingredients. However, the dose thereof may be variously prescribed by factors such as a formulation method, an administration type, age, weight, and gender of a patient, a pathological condition, food, an administration time, an administration route, an excretion rate, and response susceptibility, and those skilled in the art may suitably adjust the dose by considering the factors. The number of administrations may be one or two times within a range of clinically acceptable side effects, and even in administration sites, the composition may also be administered to one site or two or more sites. Even in the animals other than the human, the same dose as that of the human per kg may be administered, or an amount obtained by converting the dose by, for example, a volume ratio (for example, an average value) in ischemic organs (heart and the like) between target animal and human and the like may be administered. Examples of the animal to be treated according to the present invention may include humans, and mammals for other purposes, and particularly, include humans, monkeys, mice, rats, rabbits, sheep, cows, dogs, goats, horses, pigs, and the like.

Further, the present invention provides a pharmaceutical composition for prevention or treatment of neurodegenerative diseases or neurological diseases or degenerative muscular diseases or muscular diseases comprising a cell transformed with a vector including genes encoding hemagglutinin neuraminidase (HN) and fusion (F) proteins as an active ingredient.

In the present invention, the cell are the same as the description of the cell transformed with the vector including genes encoding hemagglutinin neuraminidase (HN) and fusion (F) proteins, and thus, the detailed description thereof refers to the above contents. In addition, the diseases, the administration method and the dose thereof, and the like are described the same as the administration method and the dose of the pharmaceutical composition including the vector, and thus, the detailed description thereof refers to the above contents.

Meanwhile, the present invention confirmed that the cells overexpressing the HN/F proteins promote cell fusion with other cells by the HN/F proteins, a normal gene is transferred and expressed through fusion with the damaged cells, and the expression of the gene for restoring the cell damage is regulated. In addition, the present invention confirmed that in various disease models related to cell damaged, the cells overexpressing the HN/F proteins are fused with damaged cells or cells induced gene abnormality, thereby recovering to normal cells. Therefore, the cell transformed with the vector including the genes encoding the HN/F proteins may be usefully used as an active ingredient of composition for prevention or treatment of neurodegenerative diseases or neurological diseases or degenerative muscular diseases or muscular diseases.

Further, the present invention provides a method for preventing or treating neurodegenerative diseases or neurological diseases or degenerative muscular diseases or muscular diseases comprising: administering a therapeutically effective dose of a vector including genes encoding hemagglutinin neuraminidase (HN) and fusion (F) proteins to a subject.

Further, the present invention provides a method for preventing or treating neurodegenerative diseases or neurological diseases or degenerative muscular diseases or muscular diseases comprising: administering a therapeutically effective dose of a cell transformed with a vector including genes encoding hemagglutinin neuraminidase (HN) and fusion (F) proteins to a subject.

In the present invention, the vector or the cell transformed with the vector, the administration method and the dose thereof, and the like are described the same as those of the vector including genes encoding hemagglutinin neuraminidase (HN) and fusion (F) proteins, the cells transformed with the vector, and the administration method and the dose of the pharmaceutical composition including the same, and thus the detailed description will be based on the contents.

In the present invention, the subject is an subject suffering from neurodegenerative disease, neurological disease, degenerative muscular diseases, or muscular diseases and more specifically includes mammals, for example, humans, monkeys, mice, rats, rabbits, sheep, cattle, dogs, goats, horses, and pigs of which the cell damage caused by the disease, for example, nerve cell damage or muscle cell damage can be alleviated and/or restored by administering the vector containing a gene encoding hemagglutinin neuraminidase and F protein described above or a cell transduced with the vector thereto.

The neurodegenerative diseases or neurological diseases may cause nerve cell damage, spinal nerve damage, peripheral nerve damage or nerve cell death.

In addition, the neurodegenerative diseases or neurological diseases may divided into neurodegenerative diseases or neurological diseases that cause motor nerve cell damage or death, and neurodegenerative diseases or neurological diseases that cause brain nerve cell damage or death, spinal nerve damage, or peripheral nerve damage.

In order to improve or treat the neurodegenerative diseases or neurological diseases that cause motor nerve cell damage or death, preservation and recovery of motor nerve cells must be performed. As examples of such neurodegenerative diseases or neurological diseases, spinal muscular atrophy, Kennedy's disease (spinal bulbar muscular atrophy), amyotrophic lateral sclerosis (ALS), multiple sclerosis, primary lateral sclerosis or progressive bulbar palsy may be exemplified, but the present invention is not limited thereto.

The neurodegenerative diseases or neurological diseases that cause brain nerve cell damage or death, spinal nerve damage, or peripheral nerve damage can be prevented or treated through the recovery of peripheral nerves, the nerve cells of the damaged brain or spinal cord. As examples of such neurodegenerative diseases or neurological diseases, Alzheimer's disease (AD), dementia, multi-infarct dementia (mid) , frontotemporal dementia, dementia with Lewy bodies, mild cognitive impairment, corticobasal degeneration, Parkinson's disease (PD), depression, metabolic brain disease, multiple system atrophy (MSA), Huntington's disease, progressive supranuclear palsy (PSP), epilepsy, dentatorubropallidoluysian atrophy (DRPLA), spinocerebellar ataxia, glaucoma, Stroke, brain ischemia, post-encephalitic parkinsonism, Tourette's syndrome, restless legs syndrome or attention deficit disorders with hyperactivity may be exemplified, but the present invention is not limited thereto.

The degenerative muscular diseases or muscular diseases may cause muscle cell damage or muscle cell death. As examples of such degenerative muscular diseases or muscular diseases, myopathy, congenital myopathy, congenital muscular dystrophy, Duchenne muscular dystrophy, Becker muscular dystrophy, Limb Girdle muscular dystrophy, Facioscapulohumeral muscular dystrophy, oculopharyngeal muscular atrophy, distal muscular dystrophy, Emery-Dreifuss muscular dystrophy, Myotonic dystrophy, Barth syndrome, heart failure, sarcopenia or X-linked dilated cardiomyopathy may be exemplified, but the present invention is not limited thereto.

The present invention confirmed that the cells overexpressing the HN/F proteins promote cell fusion with other cells by the HN/F proteins, a normal gene is transferred and expressed through fusion with the damaged cells, and the expression of the gene for restoring the cell damage is regulated. In addition, the present invention confirmed that in various disease models related to cell damaged, the cells overexpressing the HN/F proteins are fused with damaged cells or cells induced gene abnormality, thereby recovering to normal cells. Therefore, the vector including the genes encoding the HN/F proteins or the cell transformed with the vector may be usefully used to prevent or treat neurodegenerative diseases or neurological diseases or degenerative muscular diseases or muscular diseases.

Further, the present invention provides use of a vector including genes encoding hemagglutinin neuraminidase (HN) and fusion (F) proteins in a pharmaceutical composition for prevention or treatment of neurodegenerative diseases or neurological diseases or degenerative muscular diseases or muscular diseases.

Further, the present invention provides use of a cell transformed with a vector including genes encoding hemagglutinin neuraminidase (HN) and fusion (F) proteins in a pharmaceutical composition for prevention or treatment of neurodegenerative diseases or neurological diseases or degenerative muscular diseases or muscular diseases.

In the present invention, the vector or the cell transformed with the vector, the administration method and the dose thereof, and the like are described the same as those of the vector including genes encoding hemagglutinin neuraminidase (HN) and fusion (F) proteins, the cells transformed with the vector, and the administration method and the dose of the pharmaceutical composition including the same, and thus, the detailed description will be based on the contents. In addition, the diseases are described the same as the pharmaceutical composition for preventing or treating neurodegenerative diseases or neurological diseases or degenerative muscular diseases or muscular diseases, and thus, the detailed description will be based on the contents.

The present invention confirmed that the cells overexpressing the HN/F proteins promote cell fusion with other cells by the HN/F proteins, a normal gene is transferred and expressed through fusion with the damaged cells, and the expression of the gene for restoring the cell damage is regulated. In addition, the present invention confirmed that in various disease models related to cell damaged, the cells overexpressing the HN/F proteins are fused with damaged cells or cells induced gene abnormality, thereby recovering to normal cells. Therefore, the vector including the genes encoding the HN/F proteins or the cell transformed with the vector may be usefully used to prevent or treat neurodegenerative diseases or neurological diseases or degenerative muscular diseases or muscular diseases.

Hereinafter, the present invention will be described in detail by the following Examples. However, the following Examples just exemplify the present invention, and the contents of the present invention are not limited to the following Examples.

### Example

### Cell culture

Human adipose tissue-derived mesenchymal stem cells (hATMSCs) obtained by agreement and understanding of the K-STMECELL institutional review board (IRB) were cultured in a RKCM culture medium (added with 10% FBS and provided from the K-STEMCELL) added with antibiotics. A mouse motorneuron cell line (NSC34 Motor Neuron-Like Hybrid Cell line, CEDARLANE, USA) was cultured in a Dulbecco's Modified Eagle's medium (DMEM) (added with 10% FBS) added with antibiotics. A mouse neuroblastoma cell line (N2A cell line, ATCC) was cultured in an Eagle's Minimum Essential medium (EMEM) (added with 10% FBS) added with antibiotics. A human cervical cancer cell line (HeLa cell line, ATCC) was cultured in an Eagle's Minimum Essential medium (EMEM) (added with 10% FBS) added with antibiotics. A mouse muscle myoblast cell line (C2C12 muscle myoblast cell line, ATCC) was cultured in a DMEM culture medium (added with 10% FBS) added with antibiotics. A rat cardiomyoblast cell line (H9c2 heart myoblast cell line, ATCC) was cultured in a DMEM culture medium (added with 10% FBS) added with antibiotics.

Further, human induced pluripotent stem cells (iPSCs) prepared by receiving blood from a normal person with the consent were cultured in a state of being suspended in a culture plate containing hES cell culture medium (ThermoFisher) not containing bFGF for 4 days. Thereafter, the human induced pluripotent stem cells (iPSCs) were cultured in hES cell culture medium containing retinoic acid (RA, 5 x 10⁻⁷ M) for 4 days and cultured in a neurobasal medium [sera-free DMEM/F12 (1:1) culture medium containing EGF (20 ng/ml) , bFGF (10 ng/ml) , B27 (2%), LIF (10 ng/ml) , and heparin sodium (2 µg/ml) ] for 7 days. Suspended cells which had been separated from the aggregated colonies were cultured in a poly-1-ornithine (10 mg/ml) /laminin (5 µg/ml) -coated culture plate containing neurobasal medium for 7 days, treated with accutase every 5 to 7 days, and subcultured to obtain human induced pluripotent stem cells-derived neural stem cells (iPSCs-NSCs).

All of the cells were cultured at 37°C under 5% CO₂ supply.

### Example 1. Analysis of apoptosis inhibition effect in motorneuron cell line by cell fusion between dying motorneuron cell line and adipose-derived mesenchymal stem cells

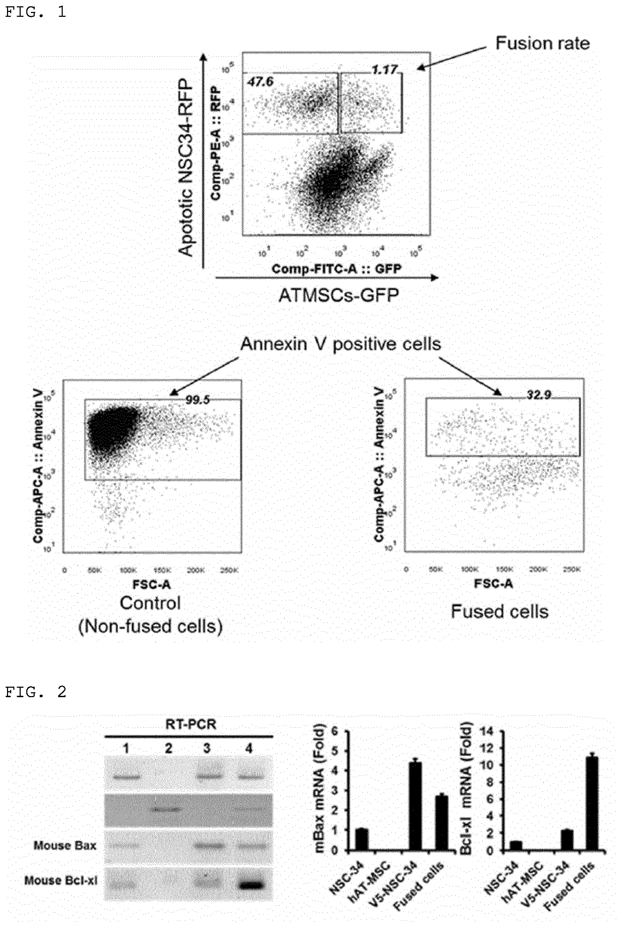

Motorneuron cell line NSC34 cells marked with a cell tracker CM-DiI (Thermofisher) were treated with 2.5 µM of thapsigargin as an apoptosis inducer and then cultured for 24 hours. After Annexin V staining, annexin V-positive NSC34 cells were isolated using an annexin V antibody by a flow cytometry. hATMSCs and annexin V-positive NSC34 cells marked with a green dye (cell-stalkerTM, Biterials) were fused using a Sendai virus (HVJ) envelope cell fusion kit (GenomONETM-CF EX Envelope Cell Fusion Kit, COSMO BIO, Japan) according to a manufacturer's method, and then V-positve cells were analyzed using a flow cytometry. As shown in FIG. 1, 99.5% of annexin V-positive cells were found in a group of NSC34 cells which were not fused with the hATMSCs, and 32.9% of annexin V-positive cells were found in a group of NSC34 cells which are fused with the hATMSCs. Such a result indicates that the death of the NSC34 motoneuron cell line may be inhibited by the cell fusion with the hATMSCs.

Further, the apoptosis inhibition effect was confirmed at a molecular level. In summary, the NSC34 cell group which was fused with the hATMSCs was isolated through the flow cytometry and then expression of a pro-apoptotic gene Bax and an anti-apoptotic gene Bcl-xL was analyzed by reverse transcription polymerase chain reaction (RT-PCR) and quantitative RT-PCR. As shown in FIG. 2, as compared with the NSC34 cell group which was not fused with the hATMSCs, in the NSC34 cell group which was fused with the hATMSCs, the expression of Bax mRNA was significantly decreased and the expression of Bcl-xL mRNA was significantly increased.

Such a result indicates that apoptosis is inhibited through the decrease in expression of the pro-apoptotic gene Bax and the increase in expression of the anti-apoptotic gene Bcl-xL when the dying NSC34 motorneuron cell line was fused with the hATMSCs.

### Example 2. Preparation of human adipose-derived mesenchymal stem cells (HN/F-hATMSCs) overexpressing hemagglutinin neuraminidase (HN)/Fusion (F) proteins

Genes of hemagglutinin neuraminidase (HN) and F protein (F) were amplified by PCR using a Sendai virus genome as a template and a primer shown in Table 1. The amplified DNAs were inserted into a pcDNA3.1 expression vector, respectively, and cloned in E. coli stain DH5α to confirm a sequence (the result was not shown). The cloned HN and F proteins are the same as published sequences GenBnak Accession No.AAB06288.1 and AAC82300.1, respectively.

**[Table 1] Primer sequence used in cloning of HN and F genes used in the present invention**

| Target gene | Primer sequences | Product size (bp) |
|---|---|---|
| HN FP | 5′-AAGCTTATGGAACAAAAACTCATCTCAGAAGAGGAT CTGGATGGTGATAGGGGCAAACGTGACTCGTACTGG-3′ | 1731 |
| HN RP | 5′-GAATTCTCATCTTTTCTCAGCCATTGCATCAAACCC ACC-3′ | |
| F FP | 5′-AAGCTTATGCATCATCATCATCATCATACAGCATAT ATCCAGAGATCACAGTGCATCTC-3′ | 1704 |
| F RP | 5′-GAATTCTCATCTTTTCTCAGCCATTGCATCAAACCC ACC-3′ | |
| Partial HN FP | 5'-CGATCTCTGGATGTGTTAG -3' | 411 |
| Partial HN RP | 5'-CCACACTAGGGTATAATGC -3' | |
| Partial F FP | 5'-CTCATGATAACTGTGGACTC -3' | 402 |
| Partial | 5'-GGTTCAGTAGGCTCTTATAC-3' | |
| F RP | | |
| Human GAPDH FP | 5'-AGAAGGCTGGGGCTCATTTG-3' | 198 |
| Human GAPDH RP | 5'-AGGGGCCATCCACAGTCTTC-3' | |

The clone obtained after cloning was amplified and cultured in an LB liquid medium for 24 hours, and a plasmid was extracted using a plasmid extraction kit (Midiprep kit) (Invitrogen, USA), and then transduced into hATMSCs using a liposome (lipofectamine 3000, Invitrogen) in a manufacturer's method to obtain hATMSCs (HN/F-hATMSCs) into which the HN/F genes were introduced. Total RNAs were extracted from the transduced cell line and the expression of the HN and F genes was analyzed by RT-PCR and quantitative RT-PCR. The hATMSCs were used as a control group. Partial HN and F primers were used for confirming expression of HN or F mRNA using RT-PCR and quantitative RT-PCR.

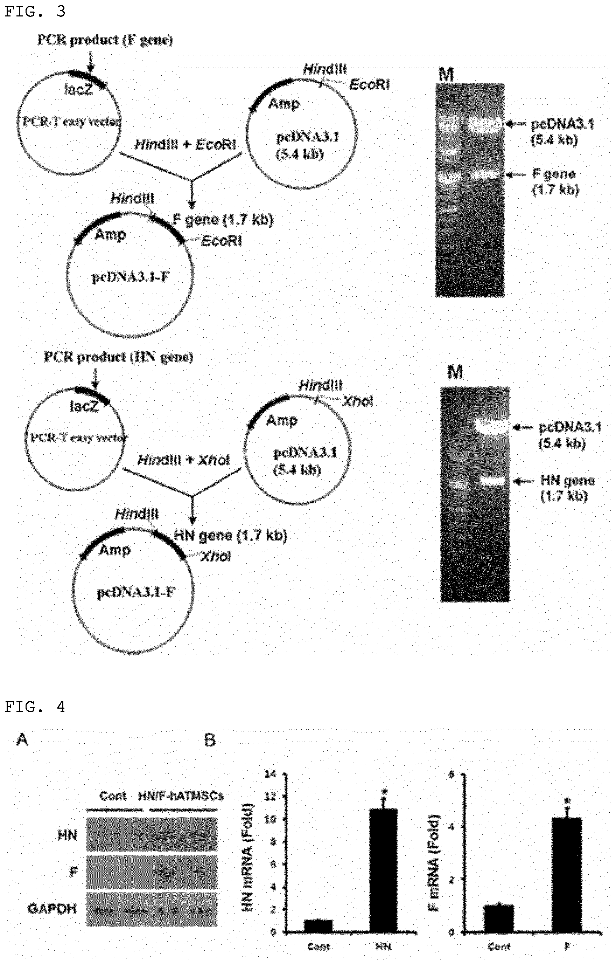

As the expression analysis result, as shown in FIG. 4, the overexpression of the HN and F genes was confirmed. Such a result indicates that the HN and F genes are successfully overexpressed through liposomal transfection at the same time.

In addition, as shown in Table 1, a base sequence encoding a myc protein was attached to a forward primer for amplifying the HN gene and a base sequence encoding histidine was attached to a forward primer for amplifying the F gene. Thus, the expression of the HN and F proteins was analyzed in the HN/F-hATMSCs using antibodies against the myc protein and the histidine (Santcruz Biotechnology, USA) by a confocal laser microscope (Nicon, Japan).

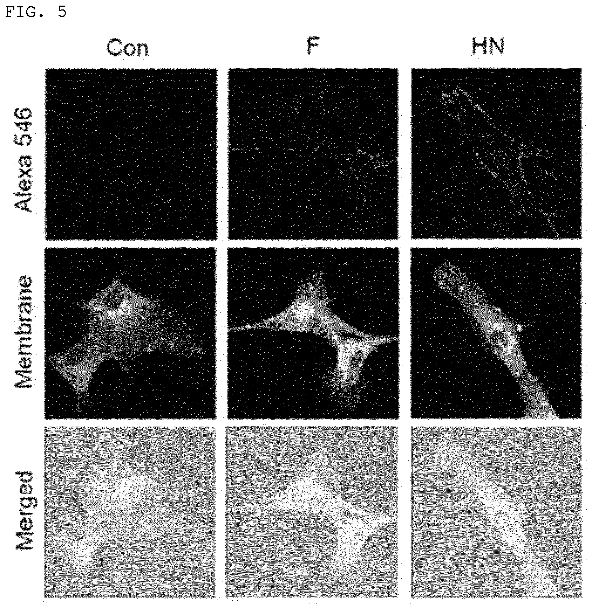

As a result of image analysis, as shown in FIG. 5, it was confirmed that the HN and F proteins were expressed on a cell surface of HN/F-hATMSCs. These results indicate that the human adipose-derived mesenchymal stem cells overexpressing the HN and F proteins are successfully prepared.

### Example 3. Evaluation of expression of stem cell markers in HN/F-hATMSCs

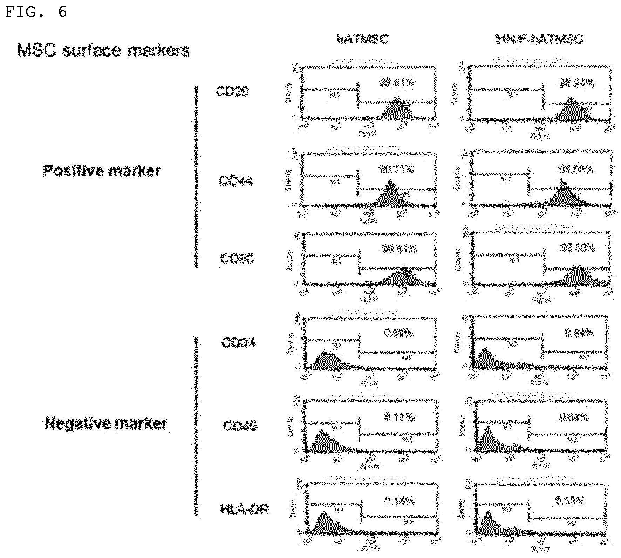

The expression for adipose-derived mesenchymal stem cell markers in HN/F-hATMSCs was analyzed using antibodies (Santcruz Biotechnology, USA) against positive markers CD29, CD44 and CD90 and negative markers CD34, CD45 and HLA-DR of the adipose-derived mesenchymal stem cells by a flow cytometry. The hATMSCs were used as a control group. As a result of expression analysis, as shown in FIG. 6, it was confirmed that there was no difference in expression between the positive markers CD29, CD44 and CD90 and the negative markers CD34, CD45 and HLA-DR as compared with the control group. These immunophenotyping results indicate that the hATMSCs introduced with the HN and F genes maintain properties of the stem cells.

### Example 4. Evaluation of cell fusion ability of HN/F-hATMSCs and NSC34 motorneuron cell line

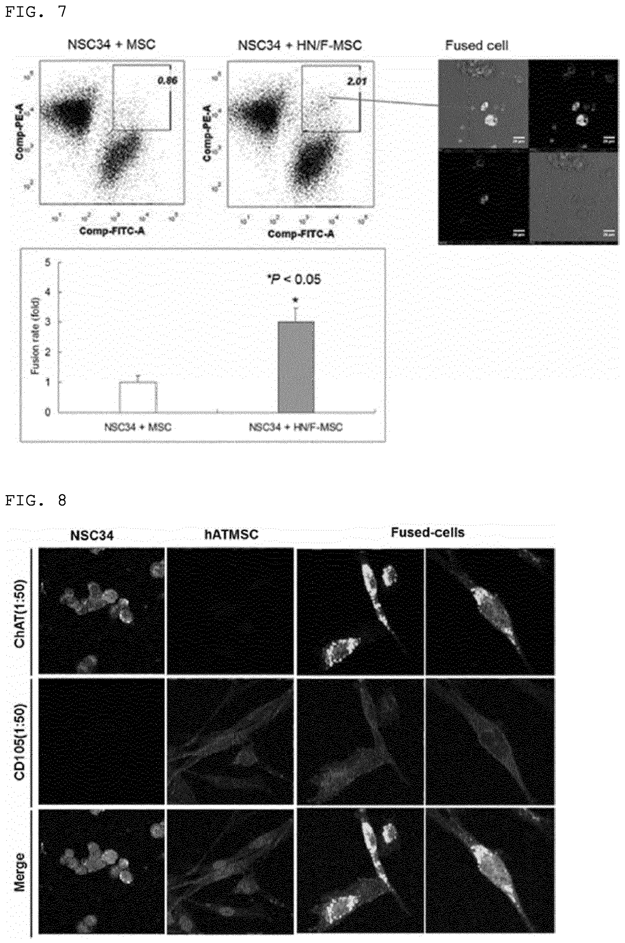

In order to evaluate the cell fusion ability of the HN/F-hATMSCs prepared in Example 2, a fusion assay of the HN/F-hATMSCs marked with a green dye and a NSC34 motoneuron cell line marked with a cell tracker CM-DiI was performed. Two types of cells were mixed in a 1.5 ml test tube at a cell number ratio of 1:1, reacted at 4°C for 5 minutes, and then reacted in a 37°C cell incubator for 15 minutes. The cells were transferred to a 6-well plate containing a DMEM culture medium (added with 10% FBS) added with antibiotics and then cultured at 37°C under 5% CO₂ supply for 16 hours. The cells were collected and a cell fusion rate of the HN/F-hATMSCs and the NSC34 motoneuron cell line was analyzed using a flow cytometry. The hATMSCs were used as a control group. As a result, as shown in FIG. 7, it was confirmed that the cell fusion rate of the HN/F-hATMSCs and the NSC34 motoneuron cell line was increased by 3.5 times or more compared to the control group. Such a result indicates that the cell fusion rate of the HN/F-hATMSCs and the NSC34 motoneuron cell line is increased.

In addition, an image of fused cells of the HN/F-hATMSCs showing green fluorescence and the NSC34 motorneuron cell line showing red fluorescence may be confirmed by an image analysis through a confocal laser microscope, as shown in FIG. 7.

### Example 5. Expression analysis of marker of hATMSCs and marker of motorneuron cells in fused cells of HN/F-hATMSCs and NSC34 motorneuron cell line

For the fused cells of Example 4, the expression of adipose-derived mesenchymal stem cell markers ChAT and CD105 was analyzed by a confocal laser microscope using antibodies (Santcruz Biotechnology, USA) against choline acetyltransferase (ChAT) as an motoneuron cell marker and an adipose-derived mesenchymal stem cell marker CD105. As a result, as shown in FIG. 8, the expression of the motoneuron cell marker ChAT and the adipose-derived mesenchymal stem cell marker CD105 was confirmed in the fused cells of the HN/F-hATMSCs and the NSC34 motorneuron cell line.

These results confirm that the HN/F-hATMSCs and the NSC34 motorneuron cell line were fused, and indicate that the fused cells express all of the marker proteins expressed in the adipose-derived mesenchymal stem cells and the motorneuron cell line.

### Example 6. Proteomics analysis of dying NSC34 motorneuron cell line and fused cells of HN/F-hATMSCs and NSC34 motorneuron cell line

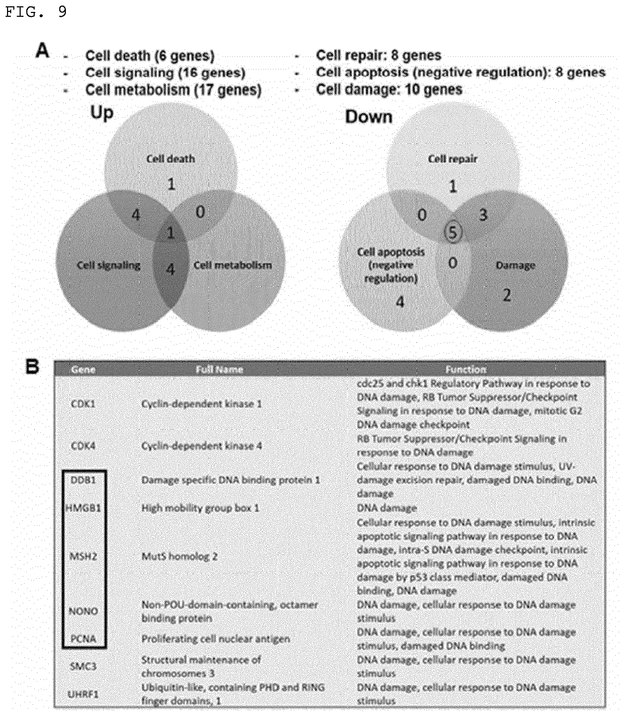

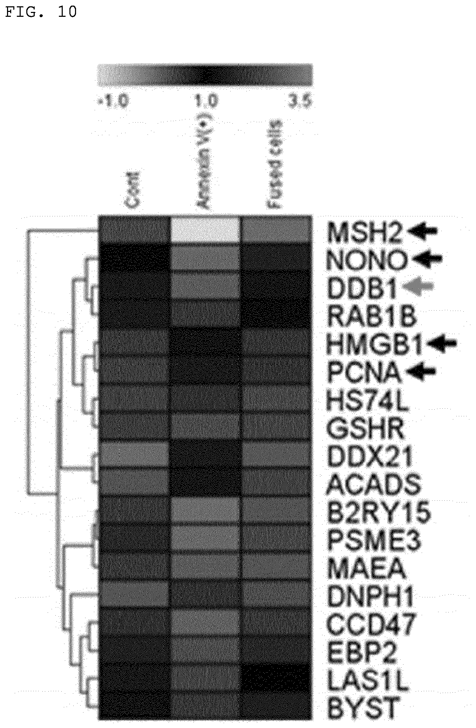

In order to find a cell damage repair mechanism through cell fusion of dying motorneuron cells and HN/F-hATMSCs, a proteomics analysis for the annexin V-positive NSC34 motorneuron cell line in Example 2 and the fused cells in Example 4 was performed using ESI-LTQ-Orbitrap (Termo Fiher) and nanoHPLC (RSLC, Dionex), and a heatmap analysis was performed using MeV software. The NSC34 motorneuron cell line was used as a control group. As the analysis result, as shown in FIGS. 9 and 10, it was confirmed that five genes (DDB1, HMGB1, MSH2, NONO and PCNA) related with cell repair were decreased in the dying NSC34 motorneuron cells, but increased in the fused cells of the HN/F-hATMSCs and the NSC34 motorneuron cell line, and thus, the five genes were selected as cell damage repair target genes.

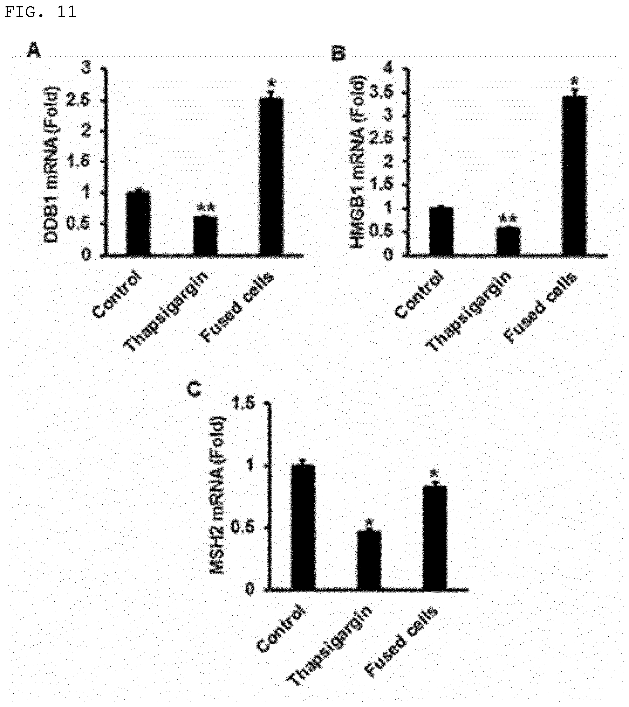

In addition, a change in expression of DDB1, HMGB1 and MSH2 among the five genes (DDB1, HMGB1, MSH2, NONO and PCNA) related with the cell repair in the annexin V-positive NSC34 motorneuron cell line of Example 2 and the fused cells of Example 4 was confirmed. In summary, the expression of DDB1, HMGB1 and MSH2 was analyzed through RT-PCR and quantitative RT-PCR in the annexin V-positive NSC34 motorneuron cell line of Example 2 and the fused cells of Example 4. The NSC34 motorneuron cell line was used as a control group. As shown in FIG. 11, the expression of DDB1, HMGB1, and MSH2 mRNAs was decreased in the dying NSC34 motorneuron cell line, but was significantly increased in the fused cells of the HN/F-hATMSCs and the NSC34 motorneuron cell line.

These results indicate that in the process in which the hATMSCs overexpressing the HN and F proteins are fused with the dying NSC34 motorneuron cell line to restore the cell damage, the DDB1, HMGB1, and MSH2 are involved.

### Example 7. Analysis of apoptosis inhibition effect in C2C12 muscle myoblast cell line by cell fusion between dying C2C12 muscle myoblast cell line and induced pluripotent stem cells-derived neural stem cells (HN/F-NSCs) overexpressing hemagglutinin neuraminidase (HN)/fusion (F) proteins

In order to find whether cells overexpressing HN and F proteins exhibit cell fusion ability, induced pluripotent stem cells-derived neural stem cells (HN/F-NSCs) that overexpress HN and F proteins were prepared. In summary, the pcDNA3.1 expression vector into which the HN and F proteins cloned in Example 2 were inserted, respectively, was transduced into induced pluripotent stem cells-derived neural stem cells (iPSCs-NSCs) using liposomes (lipofectamine 3000, Invitrogen) according to the manufacturer's method to obtain iPSCs-NSCs (HN/F-NSCs) into which HN/F genes were introduced. Total RNA was extracted from the transduced cell line, and the expression of HN and F genes was analyzed through RT-PCR and quantitative RT-PCR (results not shown).

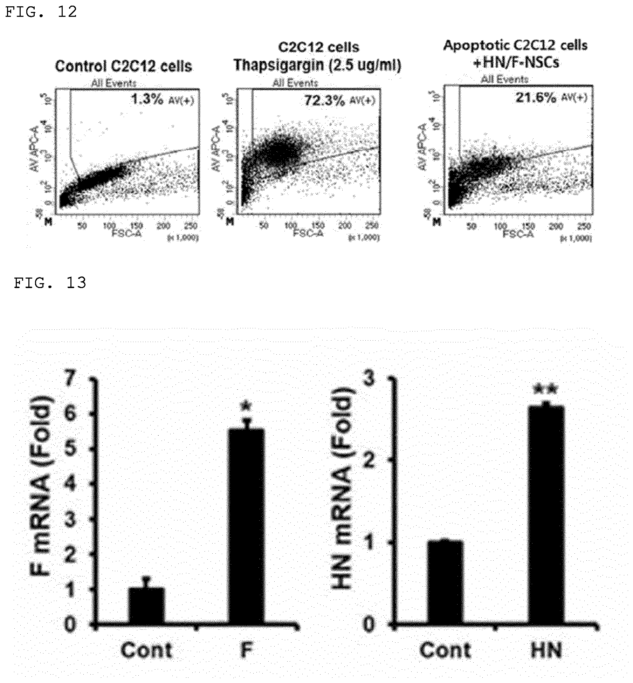

Next, a C2C12 muscle myoblast cell line labeled with a cell tracker CM-DiI was treated with 2.5 µg/ml of thapsigargin that was an apoptosis inducer and cultured for 24 hours. After annexin V staining, the annexin V-C2C12 muscle myoblast cell line was isolated through flow cytometry using an annexin V antibody. The HN/F-NSCs were labeled with a green dye, the cell fusion thereof with the annexin V-C2C12 muscle myoblast cell line was performed in the same manner as that described in Example 1, then the cells were collected, and V-positive cells were analyzed through flow cytometry. As a result of the analysis, as shown in FIG. 12, 72. 3% of annexin V-positive cells were identified in the C2C12 cell group which had not undergone cell fusion with HN/F-NSCs and 21.6% of annexin V-positive cells were identified in the C2C12 cell group which had undergone cell fusion with HN/F-NSCs.

These results indicate that iPSCs-NSCs overexpressing HN and F protein are fused with a dying C2C12 muscle myoblast cell line to restore cell damage.

### Example 8. Evaluation of cell fusion ability of N2A neuroblastoma cell line and HeLa cell line HN/F-HeLa overexpressing hemagglutinin neuraminidase (HN)/fusion (F) proteins

In order to find whether a cell fusion ability is shown even in a general cell line overexpressing HN and F proteins, a HeLa cell line HN/F-HeLa overexpressing the HN and F proteins was prepared. In summary, a pcDNA3.1 expression vector inserted with GFP-HN was cloned using a GFP-pcDNA3.1 expression vector and the HN protein gene clone obtained in Example 2. In addition, a pcDNA3.1 expression vector inserted with a RFP-F protein was cloned using a RFP-pcDNA3.1 expression vector and the F protein gene clone obtained in Example 2. Thereafter, a liposome (lipofectamine 3000, Invitrogen) was transduced into HeLa cells according to a manufacturer's method to obtain HeLa (HN/F-HeLa) introduced with HN/F genes. Total RNAs were extracted from the transduced cell line and the expression of the HN and F genes was analyzed by RT-PCR and quantitative RT-PCR. The HeLa cell line was used as a control group. As the expression analysis result, as shown in FIG. 13, the overexpression of the HN and F genes was confirmed.

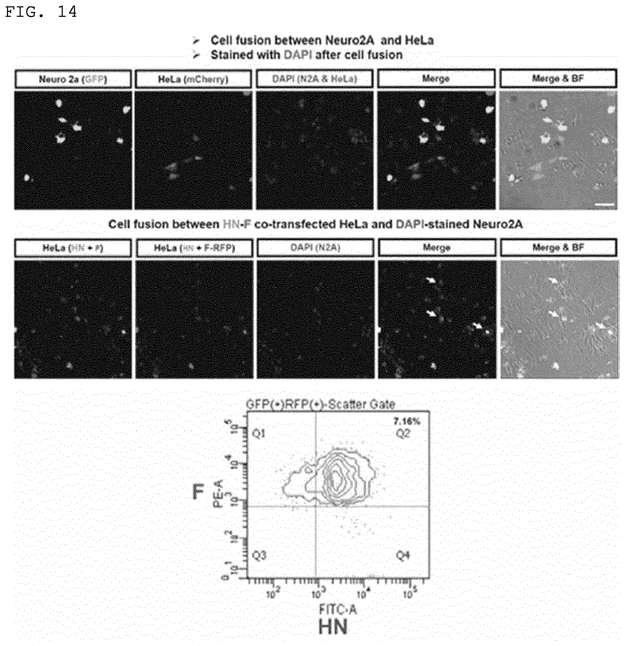

In order to evaluate the cell fusion ability of the HN/F-HeLa prepared above, the HN/F-HeLa and the N2A neuroblastoma cell line marked with the DAPI were subjected to cell fusion in the same manner as the method described in Example 4, and then the cells were collected, and the cell fusion rate of the HN/F-HeLa and the N2A neuroblastoma cell line was analyzed by a flow cytometry and a confocal laser microscope. As a control group, the N2A neuroblastoma cell line transduced with GFP and a HeLa cell line transduced with mCherry were subjected to cell fusion in the same manner as the method described in Example 4, and the cells were collected, and the cell fusion rate was compared and analyzed by a confocal laser microscope. As the analysis result, as shown in FIG. 14, it was confirmed that the cell fusion occurred in the HN/F-HeLa and the N2A neuroblastoma cell line and the cell fusion rate of the HN/F-HeLa and the N2A neuroblastoma cell line was increased by 7 times or more compared to the control group.

Such a result indicates that the cell fusion occurs by the HN and F proteins regardless of the cells.

### Example 9. Evaluation of cell fusion ability of C2C12 muscle myoblast cell line and HeLa cell line overexpressing hemagglutinin neuraminidase (HN)/fusion (F) proteins

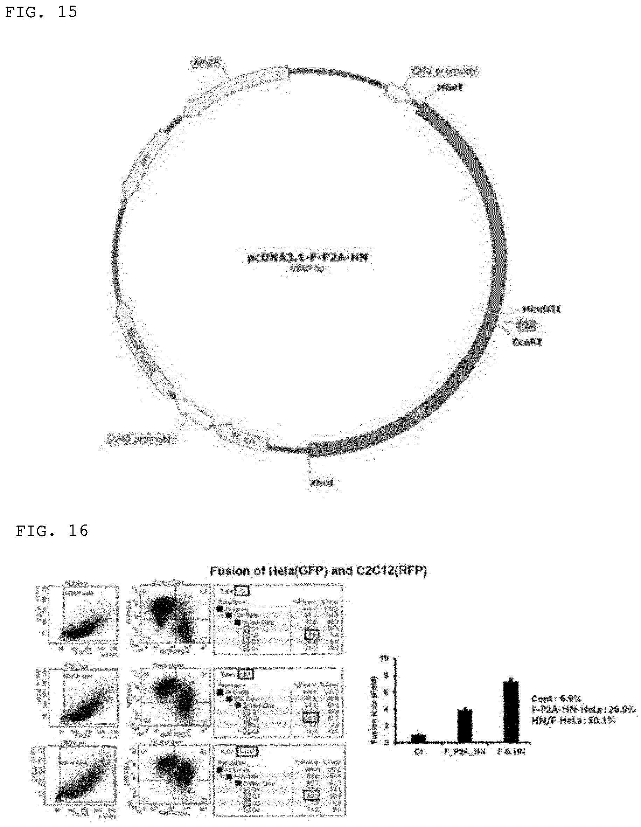

In order to evaluate the cell fusion ability of HeLa cell line overexpressing HN and F proteins through various vectors, hemagglutinin neuraminidase (HN) and fusion (F) protein genes were introduced into HeLa cell line using the pcDNA3.1 expression vector or the pcDNA3.1-P2A expression vector. In summary, the pcDNA3.1-P2A expression vector into which HN and F protein were inserted was cloned using the pcDNA3.1-P2A expression vector and the HN protein gene and F protein gene clone obtained in Example 2 as illustrated in FIG. 15. Next, the cloned pcDNA3.1-P2A expression vector was transduced into the HeLa cell line using a liposome (lipofectamine 3000, Invitrogen) according to the manufacturer's method to obtain a HeLa cell line (F-P2A-HN-HeLa) into which the HN/F genes were introduced. The pcDNA3.1 expression vector into which the HN and F protein cloned in Example 2 were inserted, respectively, were transduced into the HeLa cell line as described above to obtain a HeLa cell line (HN/F-HeLa) into which the HN/F genes were introduced. Total RNA was extracted from the transduced cell line, and the expression of HN and F genes was analyzed through RT-PCR and quantitative RT-PCR (results not shown).

Next, in order to evaluate the cell fusion ability, each of the HeLa cell lines obtained above was labeled with GFP and fused with the RFP-labeled C2C12 muscle myoblast cell line in the same manner as that described in Example 4, then the cells were collected, and the cell fusion rate of the HeLa cell line and the C2C12 muscle myoblast cell line was analyzed through flow cytometry. As a control, the GFP-labeled HeLa cell line and the RFP-labeled C2C12 muscle myoblast cell line were fused in the same manner as that described in Example 4, then the cells were collected, and the cell fusion rate thereof was analyzed through flow cytometry for comparison. As a result of the analysis, as shown in FIG. 16, the cell fusion rate of F-P2A-HN-HeLa was 26.9% and the cell fusion rate of HN/F-HeLa was 50.1%, and it was confirmed that the cell fusion rate increased by about 4 times and 7 times, respectively, as compared to that of the control.

These results indicate that HN and F proteins are overexpressed in cells through various vectors and that cells overexpressing HN and F proteins exhibit increased cell fusion ability with other cells.

### Example 10. Analysis of gene expression related with cell repair in fused cells of N2A neuroblastoma cell line and HeLa cell line

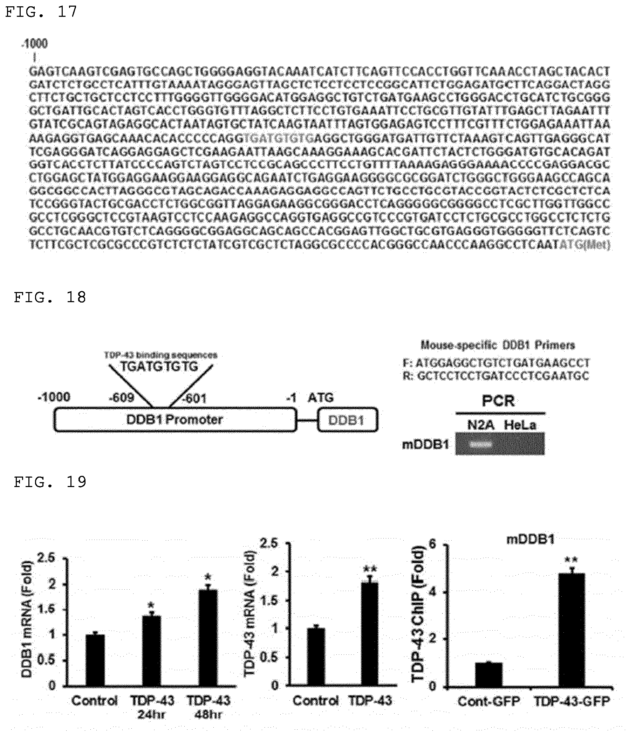

In order to confirm a cell damage repair mechanism during cell fusion by HN and F proteins, as shown in FIG. 17, it was confirmed that a binding motif of a TAR DNA-binding protein 43 (TDP-43) as a transcriptional factor (TF) was present in a promoter region of DDB1 by screening the promoter region of a gene DDB1 related with cell repair selected in Example 6.

In order to confirm that the expression of DDB1 increases by increasing TDP-43 during cell fusion by the HN and F proteins, as shown in FIG. 18, primers specifically binding to a mouse DDB1 (mDDB1) were prepared and total DNAs were extracted from the N2A neuroblastoma cell line and the HeLa cell line using the primers, and then the mouse-specific DDB1 gene was confirmed by PCR. In order to confirm that the expression of the DDB1 is regulated by the TDP-43, the TDP-43 was overexpressed in the N2A neuroblastoma cell line and then RNAs were extracted and analyzed using quantitative RT-PCR. Thereafter, the cell fusion was performed between the N2A neuroblastoma cell line and the HeLa cell line transduced with GFP and between the N2A neuroblastoma cell line and the HeLa cell line transduced with GFP-TDP-43 using a Sendai virus (HVJ) envelope cell fusion kit (GenomONETM-CF EX Sendai virus (HVJ) Envelope Cell Fusion Kit, COSMO BIO, Japan) according to the manufacturer's method, respectively, and then a ChIP chromatin immunoprecipitation (ChIP) assay (Millipore) was performed using a fusion GFP antibody (Rockland) . As a result, as shown in FIG. 19, it was confirmed that mDDB1 was increased according to the fusion time in the fused cell line of the N2A neuroblastoma cell line and the HeLa cell line transduced with GFP-TDP-43, and the occupancy of TDP-43 in the mDDB1 promoter region was increased by 4 times or more, and the expression of DDB1 was regulated by increasing TDP-43.

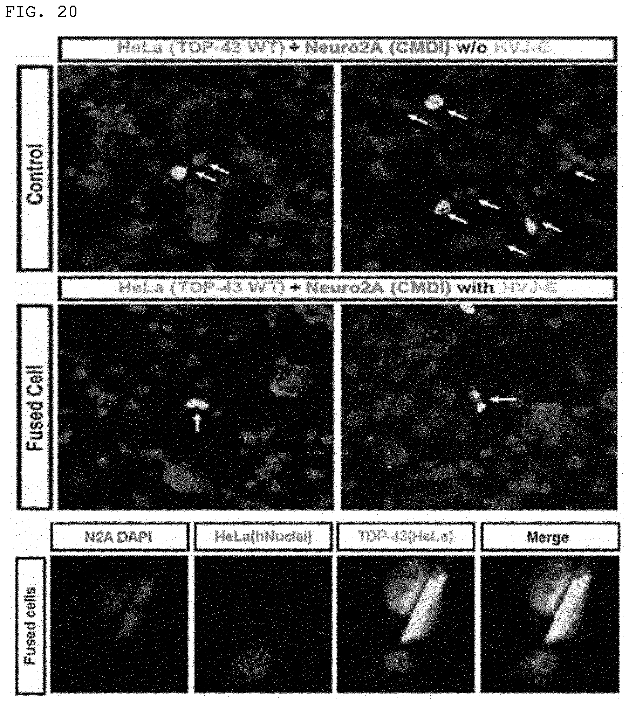

Further, in order to confirm whether the TDP-43 is translocated into a nucleus of a recipient cell during cell fusion by the HN and F proteins, the N2A neuroblastoma cell line marked with CM-DiI and the HeLa cell line transduced with GFP-TDP-43 were cell-fused in the same manner as the method described above and then translocation of TDP-43 was analyzed by a confocal laser microscope after DAPI staining. In addition, the N2A neuroblastoma cell line was stained using NucBlue (ThermoFisher) and cell-fused with the HeLa cell line transduced with GFP-TDP-43 in the same manner as the method described above and then analyzed by a confocal laser microscope after performing immunostaining using a human nuclei antibody (abeam). As the analysis result, as shown in FIG. 20, it was confirmed that the TDP-43 in the HeLa cell line transduced with GFP-TDP-43 is translocated into the nucleus of the N2A neuroblastoma cell line.

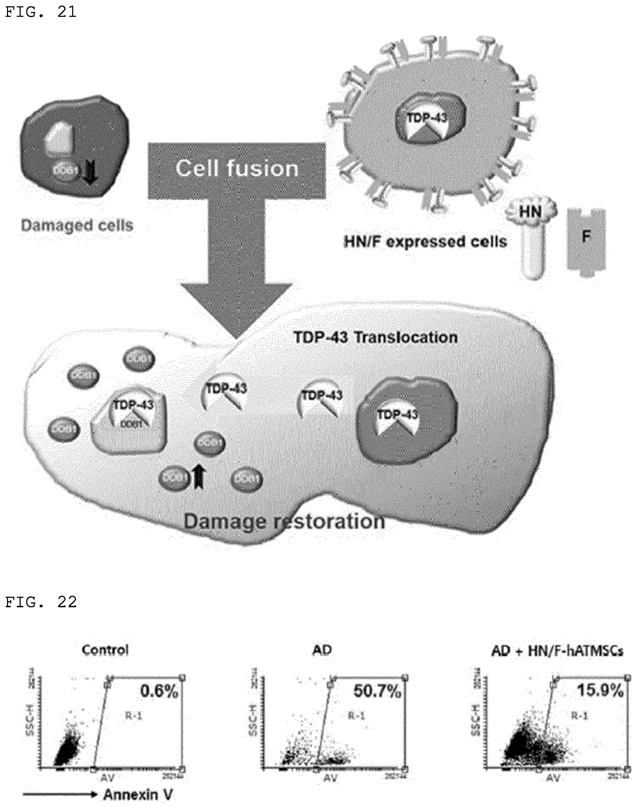

Such a result indicates that an increase in expression of the gene DDB1 related with cell repair during the cell fusion by the HN and F proteins is regulated by binding to the promoter region of mDDB1 after TDP-43 of a donor cell is translocated into the nucleus of the recipient cell. In addition, the result indicates that as shown in FIG. 21, in the cell damage-related disease, the cells overexpressing the HN/F proteins are fused with the damaged cells to translocate a transcription regulator in the cells overexpressing the HN/F proteins into the nucleus of the damaged cells and regulate the expression of the gene involved in the cell repair in the damaged cells by the translocated transcription regulator, thereby restoring the damaged cells and treating the cell damage-related diseases.

### Example 11. Analysis of therapeutic effect using cell fusion of HN/F-hATMSCs in Alzheimer's disease (AD) cell model

In order to examine the therapeutic effect using cell fusion by cells overexpressing HN/F proteins in Alzheimer's disease (AD) as a disease related to cell damage, an AD cell model was constructed. In summary, AD animal model TG2576 mouse (80 days old) was provided from Jackson laboratory. Next, the subventricular zone of the TG2576 mouse was isolated, and the stem cells were isolated from the isolated tissue and filtered using trypsin. Next, 20 ng/ml of EGF and 20 ng/ml of FGF were each added to DMEM culture medium containing B27 supplement, and the stem cells were cultured in a spherical form for 4 days. After 4 days, the stem cells were transferred to a well-plate and cultured in DMEM culture medium containing 5% FBS and B27 supplement for 7 days to induce differentiation of AD mouse-derived neural stem cells.

Next, the HN/F-hATMSCs obtained in Example 2 and the AD mouse-derived neural stem cells obtained above were fused in the same manner as that described in Example 4. After 24 hours of cell fusion, 5 µl of annexin V antibody was added thereto, the reaction was conducted at room temperature for 15 minutes, and then V-positive cells were analyzed through flow cytometry. As a result of the analysis, as shown in FIG. 22, 50.7% of annexin V-positive cells were identified in AD mouse-derived neural stem cells and 15.9% of annexin V-positive cells were identified in AD mouse-derived neural stem cells which had undergone cell fusion with HN/F-hATMSCs.

These results indicate that hATMSCs overexpressing HN and F proteins are fused with AD nerve cells by HN and F proteins to restore the cell damage seen in Huntington's disease nerve cells.

### Example 12. Analysis of therapeutic effect using cell fusion of HN/F-hATMSCs in Huntington's disease cell model

In order to examine the therapeutic effect using cell fusion by cells overexpressing HN/F proteins in Huntington's disease as a disease related to cell damage, a Huntington's disease cell model was constructed. In summary, Huntington's disease animal model R6/2 Tg mouse (80 days old) was provided from Jackson laboratory. Next, the subventricular zone of the R6/2 Tg mouse was isolated, and the stem cells were isolated from the isolated tissue and filtered using trypsin. Next, 20 ng/ml of EGF and 20 ng/ml of FGF were each added to DMEM culture medium containing B27 supplement, and the stem cells were cultured in a spherical form for 4 days. Thereafter, the stem cells were transferred to a well-plate and cultured in DMEM culture medium containing 5% FBS and B27 supplement for 7 days to induce differentiation of Huntington's disease mouse-derived neural stem cells.

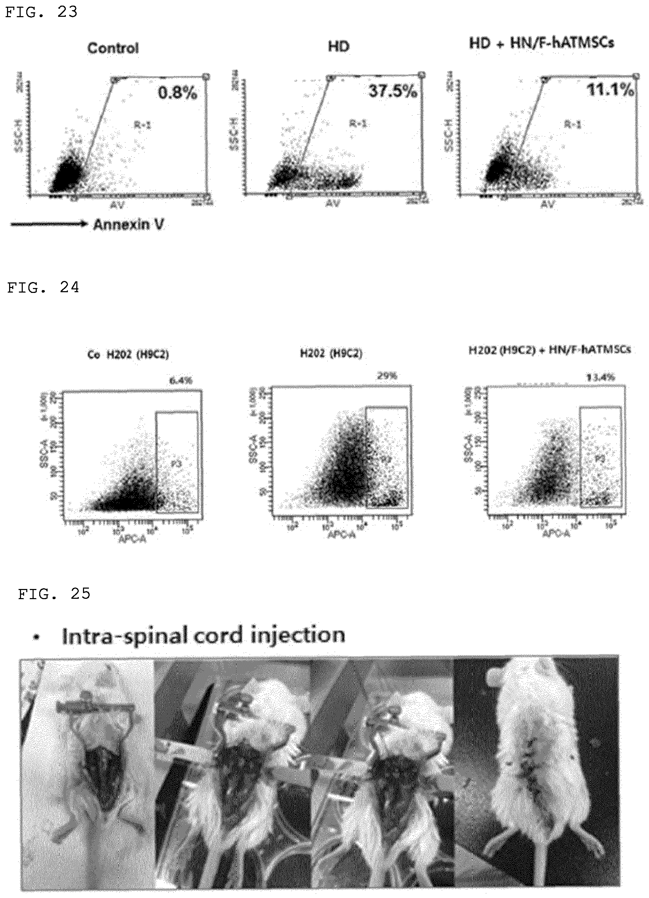

Next, the HN/F-hATMSCs obtained in Example 2 and the Huntington's disease mouse-derived neural stem cells obtained above were fused in the same manner as that described in Example 4. After 24 hours of cell fusion, 5 µl of annexin V antibody was added thereto, the reaction was conducted at room temperature for 15 minutes, and then V-positive cells were analyzed through flow cytometry. As a result of the analysis, as shown in FIG. 23 37.5% of annexin V-positive cells were identified in Huntington's disease mouse-derived neural stem cells and 11.1% of annexin V-positive cells were identified in Huntington's disease mouse-derived neural stem cells which had undergone cell fusion with HN/F-hATMSCs.

These results indicate that hATMSCs overexpressing HN and F proteins are fused with Huntington's disease nerve cells by HN and F proteins to restore the cell damage seen in Huntington's disease nerve cells.

### Example 13. Analysis of therapeutic effect using cell fusion of HN/F-hATMSCs in heart failure disease cell model

In order to examine the therapeutic effect using cell fusion by cells overexpressing HN/F proteins in heart failure disease as a disease related to cell damage, a heart failure disease cell model was constructed. In summary, cell tracker CMDFA (Invitrogen)-labeled H9c2 heart myoblast cell line was treated with 200 µM of hydrogen peroxide (H₂O₂) and then cultured for 3 hours to induce oxidative stress, whereby a heart failure disease cell model was obtained.

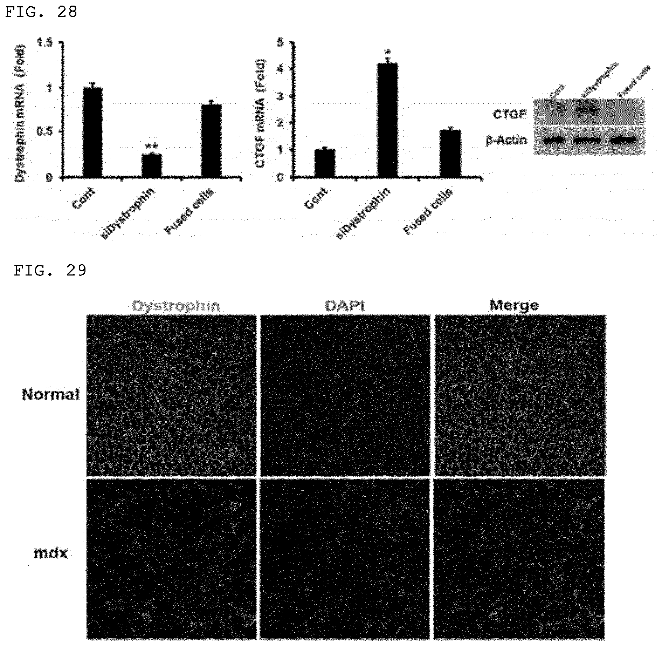

Next, the HN/F-hATMSCs obtained in Example 2 were labeled with CM-DiI and fused with the heart failure disease cell model obtained above in the same manner as that described in Example 4, and then the cells were collected. The collected cells were stained with annexin V, annexin V-positive cells were isolated through flow cytometry, and green fluorescent positive cells among the isolated cells were analyzed. As a result of the analysis, as shown in FIG. 24, 29% of annexin V-positive cells were identified in the heart failure disease cell model and 13.4% of annexin V-positive cells were identified in the heart failure disease cell model which had undergone cell fusion with HN/F-hATMSCs.

These results indicate that hATMSCs overexpressing HN and F proteins are fused with cells in which heart failure was induced by HN and F proteins to restore cell damage.

### Example 14. Analysis of therapeutic effect using HN/F-hATMSCs in amyotrophic lateral sclerosis (ALS) disease animal model

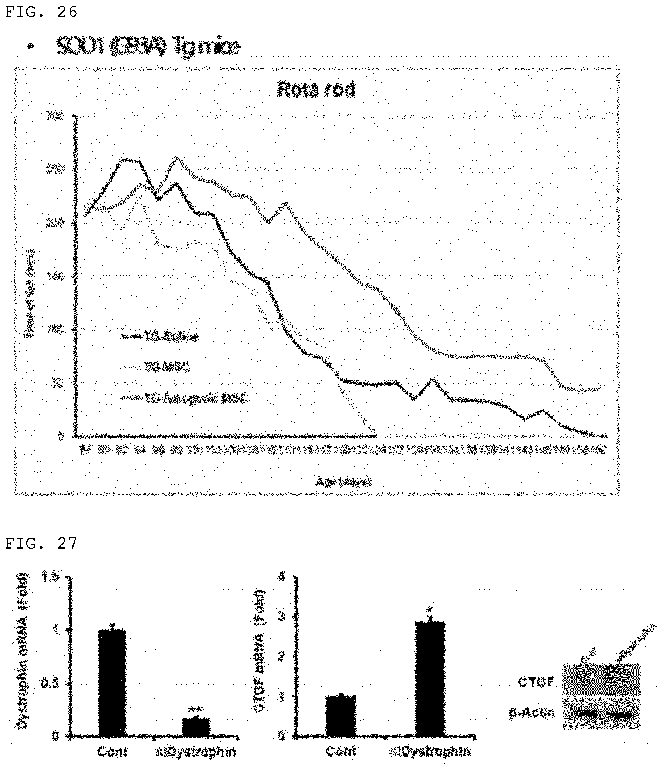

In order to confirm the therapeutic effect using HN/F-hATMSCs in an amyotrophic lateral sclerosis (ALS) disease as the cell damage-related disease, G93A SOD1 Tg mice (80 days after birth) as an amyotrophic lateral sclerosis (ALS) disease animal model were received from a Jackson laboratory. Thereafter, the G93A SOD1 Tg mice were divided into a control group (Tg-saline) , a Tg-MSC group, and a Tg-fusogenic MSC group, and as shown in FIG. 25, a saline was injected in the control group, hATMSCs were injected in the Tg-MSC group, and the HN/F-hATMSCs prepared in Example 2 were injected in the Tg-fusogenic MSC group with the cell number of 0.5 to 2×10⁶ by using an intra-spinal cord injection, respectively, and then a rota rod test was performed. As a result, as shown in FIG. 26, it was confirmed that in the Tg-fusogenic MSC group injected with the HN/F-hATMSCs, mobility was improved by the fusion of the HN/F-hATMSCs and the neuron cells by the HN/F proteins and the neuron cell damage repair.

### Example 15. Analysis of therapeutic effect using HN/F-hATMSCs Duchenne muscular dystrophy (DMD) disease cell model

In order to confirm the therapeutic effect using HN/F-hATMSCs in a Duchenne muscular dystrophy (DMD) disease as the cell damage-related disease, DMD disease cell model was prepared. In summary, DMD disease is a muscle disease caused by dystrophin deficiency in muscle fibers, and thus siRNA (siDystrophin, GenePharma) specific to dystrophin was obtained and introduced into C2C12 muscle myoblast cells according to the manufacturer's method to prepare a C2C12 muscle myoblast cell line with suppressed dystrophin expression. In order to examine the expression of dystrophin and CTGF as a pathologic symptom of DMD disease, a primer which specifically binds to dystrophin and CTGF was prepared. Total RNA was extracted from the siDystrophin-introduced C2C12 muscle myoblast cell line, and the expression of dystrophin and CTGF genes was analyzed through quantitative RT-PCR. CTGF protein expression was analyzed through western blot using CTGF antibody (Abeam). A C2C12 cell line was used as a control. As a result of the expression analysis, as shown in FIG. 27, it was confirmed that the dystrophin gene expression decreased in the siDystrophin-introduced C2C12 muscle myoblast cell line as a DMD disease cell model and the expression of CTGF gene and protein increased.

In order to examine the therapeutic effect using cell fusion by cells overexpressing HN/F proteins in the DMD disease cell model prepared above, the HN/F-hATMSCs prepared in the same manner as in Example 2 were fused with the DMD disease cell model prepared above in the same manner as that described in Example 4, then the cells were collected, and the expression of dystrophin and CTGF was analyzed through quantitative RT-PCR and western blot. As a result of the analysis, as shown in FIG. 28, it was confirmed that dystrophin expression decreased and CTGF expression increased in the DMD disease cell model but dystrophin expression significantly increased and CTGF expression significantly decreased in the cells in which HN/F-hATMSCs and the DMD disease cell model were fused.

These results indicate that cells overexpressing HN and F proteins are fused with the DMD disease cell model and restoration from the cell-fused DMD disease cell model to normal cells is achieved. These results also indicate that the normal dystrophin gene is delivered to the cell-fused DMD disease cell model and the dystrophin expression is restored.

### Example 16. Analysis of therapeutic effect using HN/F-hATMSCs Duchenne muscular dystrophy (DMD) disease animal model

In order to confirm the therapeutic effect using cell fusion by cells overexpressing the HN/F proteins in a Duchenne muscular dystrophy (DMD) disease, mdx mice (2 to 4 weeks after birth) as a Duchenne muscular dystrophy (DMD) disease animal model were received from a Jackson laboratory. The skeletal muscle was isolated from the mdx mouse, and immunohistochemistry was performed using a Dystrophin antibody (abcam), and as shown in FIG. 29, it was confirmed that Dystrophin was reduced. Thereafter, the mdx mice were divided into a control group (saline), a MSC group, and a fMSC group, and as shown in FIG. 25, a saline was injected in the control group, hATMSCs marked with a green dye was injected in the MSC group, and HN/F-hATMSCs of Example 2 marked with a green dye was injected in the fMSC group with the cell number of 0.5 to 2×10⁶ by using an intra-spinal cord injection, respectively, and skeletal muscles were isolated after 1 week. Next, the skeletal muscle was stained with hNuclei antibody (abcam), and engraftment ability of the extracted skeletal muscle was analyzed under a confocal laser microscope. Further, in 3 weeks or 15 weeks after the injection, the skeletal muscle was isolated, and immunohistochemistry was performed using a dystrophin antibody (abeam). Further, in one week after the injection, the skeletal muscle was isolated, total RNA was extracted from the isolated skeletal muscle, and the CTGF gene expression was analyzed through quantitative RT-PCR.

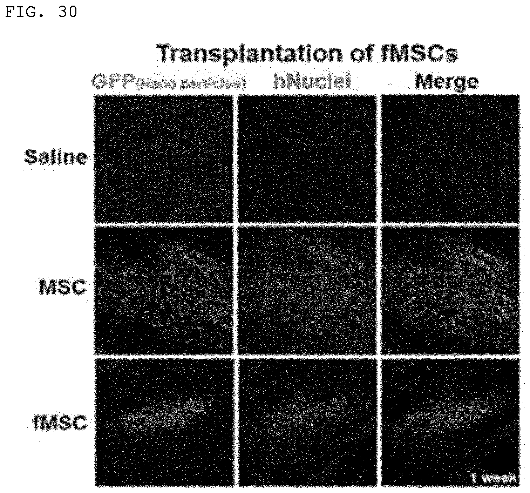

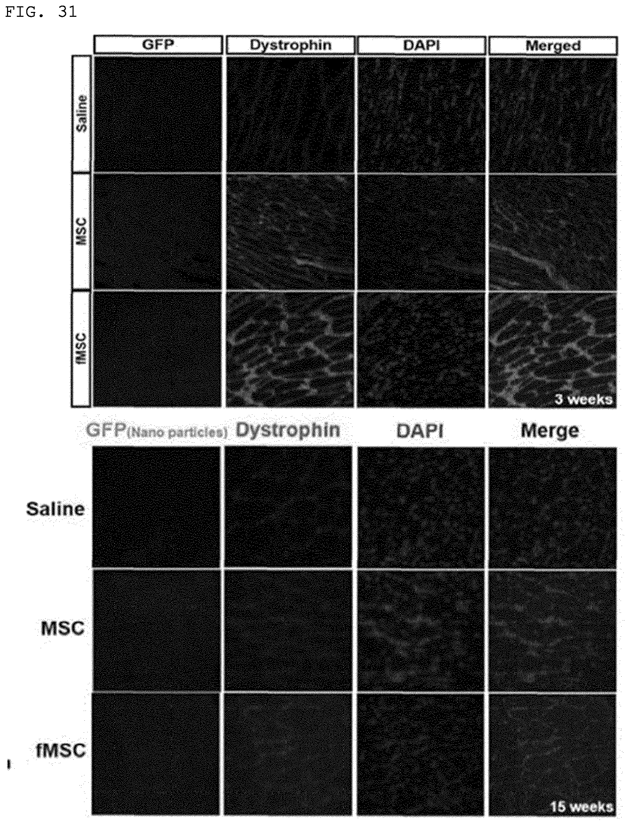

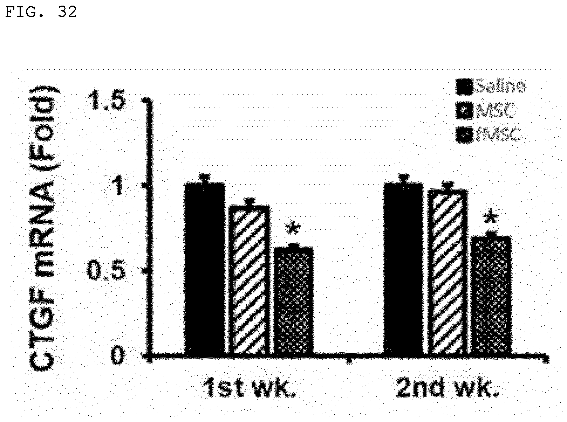

As a result, as shown in FIG. 30, the engraftment ability of hATMSCs and HN/F-hATMSCs in each of the MSC group and the fMSC group was confirmed with the green dye. As shown in FIG. 31, it was confirmed that the normal human dystrophin protein expression significantly increased in 3 weeks and 15 weeks after the injection and the dystrophin protein expression continued in the fMSC group. As shown in FIG. 32, it was confirmed that the CTGF gene expression significantly decreased in one week after the injection in the fMSC group.

These results indicate that the cell fusion of cells that normally express dystrophin with dystrophin deficient muscle cells is promoted by HN and F proteins in the muscle of mdx mouse and restoration from cell-fused muscle cells to normal muscle cells is achieved.

These results indicate that the cells overexpressing the HN/F proteins are fused with the damaged cells to translocate a transcription regulator in the cells overexpressing the HN/F proteins into the nucleus of the damaged cells and regulate the expression of the gene involved in the cell repair in the damaged cells by the translocated transcription regulator, thereby restoring the damaged cells, and thus, cell fusion technology using HN and F proteins can treat neurodegenerative diseases or neurological diseases, degenerative muscular diseases or muscle diseases, which are related to cell damage. In addition, these results indicate that since the cells overexpressing the HN/F proteins are fused with cells having gene abnormality by HN/F proteins, and normal genes can be transferred and expressed by cell fusion, the cell fusion technology using HN and F proteins may be used as a delivery tool for gene therapy.

### [Industrial Applicability]

The present invention relates to gene and cell therapy product using a cell fusion technology and uses thereof, and more particularly, cell fusion technology using hemagglutinin neuraminidase (HN) and fusion (F) proteins can be used to restore cell damage and introduce normal genes, and thus, a vector including genes encoding HN and F proteins or cells transformed therewith can be usefully used in the treatment of cell damage-related diseases, more specifically neurodegenerative diseases or neurological diseases or degenerative muscular diseases or muscle diseases.

## Claims

1. A pharmaceutical composition for prevention or treatment of neurodegenerative diseases or neurological diseases or degenerative muscular diseases or muscular diseases comprising a vector including genes encoding hemagglutinin neuraminidase (HN) and Fusion (F) proteins as an active ingredient.

2. The pharmaceutical composition for prevention or treatment of neurodegenerative diseases or neurological diseases or degenerative muscular diseases or muscular diseases according to claim 1, wherein the hemagglutinin neuraminidase and F proteins are derived from sendai virus, human immunodeficiency virus 1, Human parainfluenza virus, Human respiratory syncytial virus (HRSV), influenza virus, or vesicular stomatitis virus.

3. The pharmaceutical composition for prevention or treatment of neurodegenerative diseases or neurological diseases or degenerative muscular diseases or muscular diseases according to claim 1, wherein the vector is selected from the group consisting of a linear DNA, a plasmid DNA, a non-viral recombinant vector, a viral recombinant vector, and an inducible gene expression vector system.

4. The pharmaceutical composition for prevention or treatment of neurodegenerative diseases or neurological diseases or degenerative muscular diseases or muscular diseases according to claim 3, wherein the recombinant viral vector is selected from the group consisting of retrovirus, adenovirus, adeno-associated virus, helper-dependent adenovirus, herpes simplex virus, lentivirus, and vaccinia virus vectors.

5. The pharmaceutical composition for prevention or treatment of neurodegenerative diseases or neurological diseases or degenerative muscular diseases or muscular diseases according to claim 1, wherein the neurodegenerative diseases or neurological diseases cause motor nerve cell damage or death, brain nerve cell damage or death, spinal nerve damage, or peripheral nerve damage.

6. The pharmaceutical composition for prevention or treatment of neurodegenerative diseases or neurological diseases or degenerative muscular diseases or muscular diseases according to claim 5, wherein the neurodegenerative diseases or neurological diseases causing motor nerve cell damage or death are selected from the group consisting of spinal muscular atrophy, Kennedy's disease (spinal bulbar muscular atrophy), amyotrophic lateral sclerosis (ALS), multiple sclerosis, primary lateral sclerosis, and progressive bulbar palsy.

7. The pharmaceutical composition for prevention or treatment of neurodegenerative diseases or neurological diseases or degenerative muscular diseases or muscular diseases according to claim 5, wherein the neurodegenerative diseases or neurological diseases causing brain nerve cell damage or death, spinal nerve damage, or peripheral nerve damage are selected from the group consisting of Alzheimer's disease (AD) , dementia, multi-infarct dementia (mid), frontotemporal dementia, dementia with Lewy bodies, mild cognitive impairment, corticobasal degeneration, Parkinson's disease (PD), depression, metabolic brain disease, multiple system atrophy (MSA), Huntington's disease, progressive supranuclear palsy (PSP), epilepsy, dentatorubropallidoluysian atrophy (DRPLA), spinocerebellar ataxia, glaucoma, Stroke, brain ischemia, post-encephalitic parkinsonism, Tourette's syndrome, restless legs syndrome, and attention deficit disorders with hyperactivity.

8. The pharmaceutical composition for prevention or treatment of neurodegenerative diseases or neurological diseases or degenerative muscular diseases or muscular diseases according to claim 1, wherein the degenerative muscular diseases or muscular diseases are selected from the group consisting of myopathy, congenital myopathy, congenital muscular dystrophy, Duchenne muscular dystrophy, Becker muscular dystrophy, Limb Girdle muscular dystrophy, Facioscapulohumeral muscular dystrophy, oculopharyngeal muscular atrophy, distal muscular dystrophy, Emery-Dreifuss muscular dystrophy, Myotonic dystrophy, Barth syndrome, heart failure, sarcopenia, and X-linked dilated cardiomyopathy.

9. A pharmaceutical composition for prevention or treatment of neurodegenerative diseases or neurological diseases or degenerative muscular diseases or muscular diseases comprising a cell transduced with a vector including genes encoding hemagglutinin neuraminidase (HN) and Fusion (F) proteins as an active ingredient.

10. The pharmaceutical composition for prevention or treatment of neurodegenerative diseases or neurological diseases or degenerative muscular diseases or muscular diseases according to claim 9, wherein the cell is selected from the group consisting of a stem cell, a progenitor cell, and an animal cell.

11. The pharmaceutical composition for prevention or treatment of neurodegenerative diseases or neurological diseases or degenerative muscular diseases or muscular diseases according to claim 10, wherein the cell is selected from the group consisting of an autologous cell, an allogenic cell, and a xenogenic cell.

12. The pharmaceutical composition for prevention or treatment of neurodegenerative diseases or neurological diseases or degenerative muscular diseases or muscular diseases according to claim 10, wherein the stem cell is selected from the group consisting of an embryonic stem cell, an adult stem cell, and an induced pluripotent stem cell.

13. The pharmaceutical composition for prevention or treatment of neurodegenerative diseases or neurological diseases or degenerative muscular diseases or muscular diseases according to claim 12, wherein the adult stem cell is selected from the group consisting of a hematopoietic stem cell, a neural stem cell, and a mesenchymal stem cell.

14. The pharmaceutical composition for prevention or treatment of neurodegenerative diseases or neurological diseases or degenerative muscular diseases or muscular diseases according to claim 13, wherein the mesenchymal stem cell is a mesenchymal stem cell derived from one or more tissues selected from the group consisting of umbilical cord, cord blood, bone marrow, blood, brain, skin, fat, skeleton, muscle, nerve, periosteum, amniotic membrane, and placenta.

15. A method for preventing or treating neurodegenerative diseases or neurological diseases or degenerative muscular diseases or muscular diseases, the method comprising administering a therapeutically effective dose of a vector including genes encoding hemagglutinin neuraminidase (HN) and Fusion (F) proteins to a subject.

16. A method for preventing or treating neurodegenerative diseases or neurological diseases or degenerative muscular diseases or muscular diseases, the method comprising administering a therapeutically effective dose of a cell transduced with a vector including genes encoding hemagglutinin neuraminidase (HN) and Fusion (F) proteins to a subject.

17. Use of a vector including genes encoding hemagglutinin neuraminidase (HN) and Fusion (F) proteins in a pharmaceutical composition for prevention or treatment of neurodegenerative diseases or neurological diseases or degenerative muscular diseases or muscular diseases.

18. Use of a cell transduced with a vector including genes encoding hemagglutinin neuraminidase (HN) and Fusion (F) proteins in a pharmaceutical composition for prevention or treatment of neurodegenerative diseases or neurological diseases or degenerative muscular diseases or muscular diseases.
